# EUROPEAN PATENT APPLICATION

(11) **EP 1 251 184 A1**
(43) Date of publication of application: **23.10.2002**
(21) Application number: 00970199.6
(22) Date of filing: 30.10.2000
(51) Int. Cl.: C12S 3/12, C12M 1/40, D21C 9/10, D21C 3/00, C12N 11/14, C12N 9/12

(54) **REACTION METHOD, REACTION APPARATUS AND ENZYME**

(30) Priority: 28.10.1999 JP 30666199; 02.11.1999 JP 31302699
(71) Applicant: Kabushiki Kaisha Toyota Chuo Kenkyusho, Aichi-gun, Aichi-ken, 480-1192 (JP)
(72) Inventor: KAJINO, Tsutomu KK Toyota Chuo Kenkyusho, Aichi-gun Aichi 480-1192 (JP); TAKAHASHI, Haruo KK Toyota Chuo Kenkyusho, Aichi-gun Aichi 480-1192 (JP); SUGIYAMA, Hidehiko KK Toyota Chuo Kenkyusho, Aichi-gun Aichi 480-1192 (JP); ASAMI, Osamu KK Toyota Chuo Kenkyusho, Aichi-gun Aichi 480-1192 (JP); SASAKI, Toshiya KK Toyota Chuo Kenkyusho, Aichi-gun Aichi 480-1192 (JP); LI, Bo KK Toyota Chuo Kenkyusho, Aichi-gun Aichi 480-1192 (JP)
(74) Representative: Blumbach, Kramer & Partner GbR
(86) International application number: JP0007618
(87) International publication number: WO01031066

(57) **Abstract**

A reaction method in which a mediator in an activated state thereof is charged into a reaction system containing a substrate. Preferably, the activation of the mediator is practiced at a pre-step under mild conditions with enzymes. Further, preferably, a highly active mediator with particularly improved activity and/or stability is charged into the reaction system.

An enzymatic method for decomposing a substrate which comprises: immobilizing peroxidase as an oxidoreductase in a structure unit with structural stability and with a dimension approximately fitting to the size of the enzyme; and decomposing the substrate with an oxidizing reagent within a concentration range allowable through the immobilization.

A reaction promoter for use in a decomposition reaction system of a substrate with an oxidoreductase, the reaction promoter having a diketone structure radicalizable on the basis of the action of the oxidoreductase and being soluble in water.

A manganese peroxidase enzyme solution and manganese peroxidase obtained by culturing an SC26 strain as a mutant strain of Phanerochaete crysosporium.

## Description

### Technical Field

The present invention relates to a reaction method, a reaction apparatus and an enzyme. More specifically, the invention relates to a reaction method for decomposing a reaction substrate using an active mediator; a reaction apparatus advantageous for use in a specific embodiment of the reaction method; an enzymatic method for decomposing certain types of substrates; a reaction promoter; a method for decomposing a hydrophobic substrate or a persistent chemical utilizing the reaction promoter; novel manganese peroxidases; and a method for producing the manganese peroxidases.

### Background of the Invention

In recent years, it has been known that there exist enzymatic reactions mediated by mediators as. reaction mediating substances, for example, reactions involving oxidoreductases such as peroxidase and laccase. In other words, the reactions have a reaction mechanism such that mediators as reaction mediating substances are activated into active mediators by enzymes as mediator activating means and the active mediators then exert actions on substrates to perform predetermined reactions. These reactions can favorably be utilized for decomposition treatment of persistent chemicals, such as lignin as a substance for coloring paper pulp, and endocrine disruptors, particularly dioxin causing serious concerns because of its high toxicity. These reactions are very useful because they dispense with the use of chemical bleaches such as chlorine inducing problems during liquid waste disposal and the thermal decomposition causing significant environmental burdens. Particularly, the enzyme manganese peroxidase (referred to as MnP hereinafter) is an effective enzyme which activates a mediator divalent manganese ion into trivalent manganese ion in the presence of hydrogen peroxide. Then, the active mediator oxidizes various subjective substances through the radicalization of a reaction promoter (such as a known malonic acid).

As known techniques relating to the reactions, for example, "the Japan TAPPI (Technical Association of the Pulp and Paper Industry) Journal, vol.150, p. 59-68 (Harazono et al.)" refers to the decomposition of a coloring substance lignin contained in pulp with peroxidase, in which manganese ion works as a mediator for the decomposition reaction. Japanese Patent Application Laid-open No. 158790/1999 describes a similar lignin decomposition with laccase, in which HBT (1-hydroxybenzotriazole) works as a mediator for the decomposition reaction. Further, Japanese Patent Application Laid-open No. 239396/1997 discloses the decomposition technique of persistent chemicals using peroxidase as an oxidoreductase. According to the previous techniques described above, however, all the mediators are charged as mediators in inactive states into reaction systems (for example, a liquid containing pulp to be treated) where substrates exist, and then the mediators are activated in the reaction systems into active mediators by enzymes. Thus, the following problems have been remarked. First, when enzymes are charged in liquids to be treated, enzymes which are expensive cannot readily be separated and recovered. An approach for immobilizing such enzymes on insoluble carriers and then separating immobilized enzymes from liquids during recovery involves difficulty in practice when various solid fragments exist in mixture in the liquids to be treated. The approach for immobilizing an enzyme on an immobilization bed is poor in terms of contact efficiency between the enzyme and a specific low-concentration substance thereof. An approach for passing a liquid to be treated through a column on which an enzyme is carried is hardly applicable when solid fragments exist in mixture in the liquid to be treated. Second, industrial treatment processes demand treatment efficiency, and depending on the content of the required alternation of a substance, a high reaction efficiency is required under severe conditions, such as high temperature, a high acidity region, a high alkalinity region and the like. However, enzymes are inactivated under such severe conditions. Thus, the purpose cannot be attained. Third, a very significant item has never been examined as to whether or not a practical mediator activating means other than enzymes can exist for a reaction in which any active mediator meets its purpose if it eventually acts on the substrate. Further, no examination has been made yet about a significant item as to whether or not the activation of the mediator in the reaction system is essential. Fourth, because of the limitation that a mediator is activated by an enzyme in a reaction system, the condition or level of mediator activation (for example, the level of excitation state for mediator activation) is greatly restricted.

It is a first object of the invention to highly efficiently promote the decomposition of a substrate, particularly a persistent chemical substrate, with peroxidase as an oxidoreductase in the presence of a high concentration of an oxidizing reagent and to avoid the inactivation of the expensive enzyme to effectively recover and recycle the enzyme. It is also the object of this invention to conceive a practical mediator activating means other than enzymes and a method for effectively utilizing the mediator activating means.

As known techniques for the improvement of the stability of the enzyme, Gravski, A. C. et al. report in Appl. Biochem. Biotechnol. 60, 1-17 (1996) manganese peroxidase immobilized on an organic polymer particle, while Fawer, M. S. et al. (Biochem. Biophys. Acta., 1076, 15-22 (1991)) and Ather, M. et al. (Appl. Biochem. Biotechnol., 38, 57-67 (1993)) report lignin peroxidase immobilized on individual particles of glass bead, organic polymers and agarose. When oxidoreductases are immobilized on organic polymer particles, glass bead and agarose, the enzymes can effectively be recovered and recycled, depending on the manner of using the immobilized enzymes (column packing and the like) or the post-treatment (filtration recovery and the like). As a general merit of immobilized enzyme, the allowable range of temperature and pH for the enzyme has been believed to be enlarged relatively. According to the researches by the present inventors, however, it has been found that the allowable range of the oxidizing reagent concentration for oxidoreductases immobilized by conventional methods is hardly enlarged, compared with pre-immobilization. For the decomposition of a substrate, particularly a persistent chemical substrate, by utilizing an oxidizing reagent, it is a very important technical issue to improve the decomposition efficiency by raising the oxidizing reagent concentration as high as possible. Hence, immobilized enzymes not suited for this method have a fatal defect in practical use.

It is therefore a second object of the invention to provide a reaction promoter capable of discriminating similar substances such as lignin and cellulose for selective decomposition, and an enzymatic decomposition method utilizing the reaction promoter. The inventors have paid attention to the fact that malonic acid as a conventional reaction promoter is a hydrophilic dicarboxylic acid and therefore the resulting radical is also hydrophilic. Hydrophilic radical preferentially decomposes hydrophilic cellulose rather than hydrophobic lignin or at least simultaneously decomposes lignin and cellulose.

As known techniques concerning the reaction promoter, "the Japan TAPPI Journal, vol. 50, No. 4, 1996" indicates that malonic acid is effective as a chelating agent for stabilizing manganese ion at trivalence. According to the understanding of the inventors, malonic acid is a reaction promoter, which is radicalized with trivalent manganese ion and decomposes lignin and the like. Nevertheless, the researches of the inventors have revealed that malonate radical exerts a certain level of decomposition potency on lignin but also acts on cellulose. Therefore, pulp bleaching, namely lignin decomposition, is likely to be insufficient, involving also cellulose damage, so that paper with low folding endurance is produced. Generally speaking, it is fairly difficult to discriminate very similar polymeric substances lignin and cellulose from each other and selectively decompose one of them.

It is therefore a third object of the invention to provide a reaction promoter capable of discriminating similar substances such as lignin and cellulose for selective decomposition, and an enzymatic decomposition method utilizing the same. The inventors have paid attention to the fact that malonic acid as a conventional reaction promoter is a hydrophilic dicarboxylic acid and the radical generated is therefore hydrophilic. More specifically, the hydrophilic radical preferentially decomposes hydrophilic cellulose rather than hydrophobic lignin or at least simultaneously decomposes both lignin and cellulose.

Further, as known techniques regarding manganese peroxidase, Cai D. et al. report in J. Biotechnol., vol. 30, 79-90 (1993) that four types of MnP exist in a white rot fungus species (Phanerochaete crysosporium), and only analyze physico-chemical properties including for example isoelectric point and molecular weight for three MnP types among them. Further, T. K. Kirk et al. report in Enzyme Microbiol. Technol., vol. 18, 27-32 (1986) a mutant strain of Phanerochaete crysosporium prepared by UV irradiation of Phanerochaete crysosporium to improve the MnP generation quantity. No analysis is made about the properties of MnP generated by the mutant strain, such as thermal resistance and hydrogen peroxide resistance. Meanwhile, thermal resistance of an enzyme is generally a problem for the enzyme reaction. Because of trivalent manganese ion and the reactivity of radicals generated thereby, the reaction temperature for a reaction in which MnP is involved is desirably set high in many cases to raise the reaction efficiency. Because the efficiency of a reaction in which MnP is involved is also affected by the concentration of hydrogen peroxide, alternatively, a demand to set the concentration of hydrogen peroxide as high as possible is common. So as to satisfy these demands, MnP should have excellent thermal resistance and excellent hydrogen peroxide resistance as well. Conventionally, however, there has been reported MnP with thermal resistance enduring 50 °C for about 10 minutes, but with poor hydrogen peroxide resistance (Japanese Patent Application Laid-open No. 274958/1995). In contrast, there has been reported MnP with hydrogen peroxide resistance such that the residual activity of the MnP after one-minute exposure to hydrogen peroxide at a concentration of 10 mM or less amounts to 35 % or more (Japanese Patent Application Laid-open No. 2000-83658). However, because the MnP is continuously in contact with hydrogen peroxide, the resistance for such a short time as one minute is not effective for practical use. Further, the publication never tells definite experimental conditions for the thermal resistance of MnP. Because MnP with both of excellent thermal resistance and great hydrogen peroxide resistance for practical use has never been provided as described above, currently, the industrial utilization thereof has been insufficient despite the usefulness of MnP.

It is a fourth object of the invention to provide MnP with both of the thermal resistance and hydrogen peroxide resistance capable of satisfying the demands in practical use, and a method for producing such MnP.

### Disclosure of the Invention

A first aspect of the invention relates to a reaction method in which a mediator acting in an activated state thereof on a substrate to perform a predetermined reaction is charged as an active mediator in the activated state into a reaction system where the substrate exists. Because the mediator is activated in advance and is then charged into the reaction system, in the first aspect, the presence of mediator activating means in the reaction system is not required. Therefore, there is no difficulty of recovering the mediator activating means (for example, expensive enzyme) from the reaction system where the substrate exists in mixture, unlike the conventional techniques. Further, because no mediator activating means (for example, enzymes readily inactivated) is present in the reaction system, the reaction system can be set under severe conditions with high reaction efficiency, for example, under condition of high temperature, high acidity regions or high alkalinity regions. Furthermore, because mediators can be prepared by any optional methods or optional means with no account of the relation with the reaction system, there is much freedom in preparing the mediator activating means. Consequently, a highly active mediator with high activity and/or high stability as never realized via enzyme activation can be prepared. Further, the active mediator or highly active mediator can be stored preliminarily for the charging thereof into a reaction system. Thus, the active mediator or highly active mediator at a desired quantity can appropriately be charged in a reaction system at a desired timing, so that the reaction system can be controlled more easily.

A second aspect of the invention relates to a reaction method which comprises prearranging an activation step of activating a mediator by an enzyme as mediator activating means, and charging the resulting active mediator activated by the activation step into the reaction system. If the step of activating a mediator and a step of allowing the active mediator to react with a substrate are performed in the same reaction field, the difficulty involved in the separation and recovery of the enzyme or the problem concerning the contact efficiency between the enzyme and the substrate can not be overcome. In the second aspect of the invention, however, the activation step in the absence of any substrate is isolated from the substrate reaction step in the presence of a substrate. Therefore, the mediator activating means is initially separated from the substrate (in other words, a liquid to be treated, where various solid fragments, water-soluble substances and the like exist in mixture). Thus, the recovery of the mediator activating means of an enzyme is easy. More specifically, in the case that an enzyme as the mediator activating means is carried on an insoluble carrier in powder or granule, the enzyme can readily be recovered by simply separating the enzyme from an aqueous solution of the active mediator by an appropriate solid-liquid separation method (a method for separating solid and liquid from each other), such as filtration. In the case that the enzyme is carried for example on a solid bed, alternatively, the role of the solid bed resides in the activation of the mediator in the absence of any substrate, so no concern arises about the contact efficiency between the enzyme and a specific substrate at a low concentration. Because no extra solid fragment exists in mixture in the mediator solution, it is readily possible to improve the mediator activation efficiency by passing the mediator solution through a column carrying the enzyme. The second aspect of the invention is under a condition that an aqueous solution of the active mediator, which is generally water soluble, can be separated and transferred from the mediator activating means. As far as a fixable structure such as electrode or an enzyme immobilized on an insoluble carrier is used as the mediator activating means (provided that the mediator activating means is not a water soluble substance), the separation can be readily done by a simple transfer of the solution or a simple solid-liquid separation procedure.

More preferably, in the case that the active mediator in the second aspect is a stable substance (a stable low molecular substance, in particular), the activation step is performed allowing the enzyme and the mediator to be in contact under mild conditions, while the reaction between the active mediator and the substrate is performed under severe conditions yielding a higher reaction efficiency. This reaction method is on the premises that the mediator activating means is an enzyme and the active mediator is a stable substance. Under such premises, the activation step is effected under mild conditions and so the enzyme is not readily inactivated, while a high substrate treatment efficiency suitable for the industrial treatment can be expected because the substrate reaction step is done under severe conditions for a high reaction efficiency. Then, the active mediator as such stable substance (stable low molecular substance, in particular) hardly loses the activity even under severe reaction conditions. More specifically, the inconsistence between the demand toward high treatment efficiency and the demand toward retention of reaction activity during industrial treatment processes as seen in the conventional techniques can be avoided without difficulty.

A third aspect of the invention relates to a reaction method which comprises charging into the reaction system a highly active mediator in a highly activated state with more increased activity and/or more enhanced stability than those of an active mediator in the activated state in the first aspect. Because the highly active mediator has more increased activity and/or more enhanced stability than those of general active mediators, the reaction method in the first aspect can be performed at a higher efficiency. Further, this aspect enables to set severer reaction conditions at higher efficiencies, such as reactions at higher temperature, in a higher acidity region, in a higher alkalinity region and the like.

In the third aspect of the invention, more preferably, the highly active mediator is in a more highly active, excited state than the above-mentioned active mediator is, or the highly active mediator is a higher-order mediator compound of a mediator at least including a complex. These are typical examples of the highly active mediator. In the first to third aspects of the invention, more preferably, the active mediator or the highly active mediator is prepared by subjecting the mediator to enzymatic actions, catalytic actions, photoirradiation, electromagnetic irradiation, voltage application, or plasma preparation. Since the means for preparing the active mediator or the highly active mediator does not require the activation of mediators in reaction systems where substrates exist, it may be any appropriate means including enzyme. Typical examples utilize enzymatic actions, actions of catalysts except for enzyme, photoirradiation (including photoirradiation for photocatalyst excitation), electromagnetic irradiation, voltage application, or plasma preparation. In the first to third aspects of the invention, more preferably, the enzyme is an oxidoreductase such as peroxidase and laccase, and the substrate is lignin to be decomposed for pulp bleaching. In such case, an embodiment of the reaction method can be provided, which is highly demanded in practice.

A fourth aspect of the invention relates to a reaction apparatus for performing the reaction method in the second aspect, wherein the mediator activating means is in a form separable from the active mediator, and which further comprises an activation reaction field capable of performing the activation step, and a substrate reaction field being in communication through transfer means of the active mediator with the activation reaction field and capable of performing the reaction between the active mediator and the substrate. The reaction apparatus provides an effective approach for practicing the second aspect of the invention.

A fifth aspect of the invention relates to an enzymatic substrate decomposition method for decomposing an enzyme substrate with peroxidase an oxidoreductase using an oxidizing reagent, wherein an immobilized enzyme prepared by immobilizing the oxidoreductase in a structure unit with structural stability and with a dimension (namely, volume) approximately fitting to the size of the enzyme, is used to decompose the substrate in the presence of the oxidizing reagent within a concentration range allowed due to the immobilization. The immobilized enzyme for use in the fifth aspect of the invention is at relatively higher stability against temperature and pH, compared with conventional immobilized oxidoreductases. It has been found that the immobilized enzyme has a significantly wide allowable range against the concentration of oxidizing reagents such as hydrogen peroxide, in particular. The reason is not completely elucidated but is believed to possibly reside in those described below: the steric structure of the oxidoreductase immobilized in the structure unit is not wholly exposed to outer environment; the structure unit has a strong protective action for the oxidoreductase because of its structure stability; the structure unit is in a dimension approximately fitting to the size of the oxidoreductase, so that an appropriate protective action can be exerted for the oxidoreductase; and the like. Because the allowable concentration range for the oxidoreductase is prominently wide in the fifth aspect of the invention, first, a high concentration of the oxidizing reagent can be charged into the substrate to raise the decomposition efficiency. Second, the enzyme inactivation does not readily occur due to the elevation of the oxidizing reagent concentration, even though the precise control of the oxidizing reagent concentration is technically difficult. As a general characteristic property of immobilized enzyme, it is needless to say that the oxidoreductase can be recovered and recycled, in the case that the immobilized enzyme is used in the forms of column packing and immobilization onto an immobilization bed through which liquids to be treated pass or in the case that an immobilized enzyme charged in a liquid to be treated with no solid content is to be filtered and recovered after the termination of reaction.

In the fifth aspect of the invention, more preferably, the substrate is a persistent chemical. In that case, it is strongly demanded that the oxidizing reagent at a concentration as high as possible should be charged for the enzymatic reaction to raise the decomposition efficiency. Thus, the practical value of the fifth aspect of the invention can be exerted very well. In the fifth aspect of the invention, more preferably, the persistent chemical is lignin. The demand toward the decomposition of the coloring substance lignin for pulp bleaching is strong, particularly in industries. In the fifth aspect of the invention, more preferably, the structure unit is each pore in a mesoporous silica material. In this case, the structure unit is provided in an embodiment with a high efficiency, such that the oxidoreductase is immobilized in the numerous pores of the porous material, resulting in a great reaction efficiency. Advantageously, the structure unit of the silica material has extremely high structure stability.

A sixth aspect of the invention relates to a reaction promoter for use in a substrate decomposition reaction system with an oxidoreductase, and the reaction promoter has a diketone structure radicalizable on the basis of the action of the oxidoreductase and is also water-soluble. The reaction promoter soluble in water can be dissolved in an aqueous solution containing the oxidoreductase and is readily modified into radical via the action of the oxidoreductase. When the promoter is charged into a pulp bleaching system via oxidoreductase, thus, the promoter is effectively dissolved in a pulp solution and is additionally modified into radical to serve for lignin decomposition, so that pulp can be bleached. Additionally, the reaction promoter with such diketone structure has a chemical structure with particularly intense hydrophobicity, as a reaction promoter soluble in water. Thus, the promoter can readily be put into contact with hydrophobic lignin in pulp solution to decompose lignin, but is hardly put into contact with hydrophilic cellulose, to scarcely decompose cellulose. In other words, the promoter definitely discriminates lignin and cellulose from each other for selective decomposition. Consequently, pulp is sufficiently bleached through lignin decomposition with no damage on cellulose, resulting in the production of paper with high folding endurance. The reaction promoter in the sixth aspect of the invention is not limited to pulp bleaching, but may be used in combination with an oxidoreductase for a substrate solution where a hydrophilic substrate and a substrate hydrophobic relatively thereto exist in mixture in water, thereby to decompose the hydrophobic substrate selectively. In the sixth aspect of the invention, more preferably, the reaction promoter is acetylacetone.

A seventh aspect of the invention relates to a method for promoting the decomposition of a hydrophobic substrate, which comprises allowing an oxidoreductase, a reaction mediator, and the reaction promoter in the sixth aspect of the invention to coexist in an aqueous solution where a hydrophilic substrate and a substrate being hydrophobic relatively thereto exist in mixture, and preferentially decomposing the hydrophobic substrate with the radicalized reaction promoter. When the reaction promoter in the sixth aspect of the invention is used in combination with an oxidoreductase and a reaction mediator for a substrate solution where a hydrophilic substrate and a relatively hydrophobic substrate exist in mixture in water, as in this aspect of the invention, the radicalized hydrophobic reaction promoter decomposes the hydrophobic substrate selectively. Compared with the case using a reaction promoter mainly decomposing hydrophilic substrates or with the case using a reaction promoter equally decomposing hydrophilic and hydrophobic substrates: first, the potency of decomposing the hydrophobic substrate is therefore relatively higher; and second, the decomposition of the hydrophilic substrate can be suppressed. In other words, the method for promoting the decomposition of a hydrophobic substrate in accordance with the seventh aspect of the invention is extremely effective when it is desired to decompose sufficiently the hydrophobic substrate in a substrate solution as described above, particularly when it is desired to avoid the decomposition of any hydrophilic substrate. The general reaction mechanism of the seventh aspect of the invention follows a mechanism such that the reaction mediator is first activated (for example, manganese is activated into trivalent manganese ion) with an oxidoreductase, and the activated reaction mediator radicalizes a reaction promoter, which then decomposes such substrate.

In the seventh aspect of the invention, more preferably, the method for promoting the decomposition of a hydrophobic substrate comprises activating a reaction mediator with an oxidoreductase and adding the activated reaction mediator and the reaction promoter in the sixth aspect of the invention to an aqueous solution containing a hydrophilic substrate and a relatively hydrophobic substrate in mixture, to preferentially decompose the hydrophobic substrate with the reaction promoter radicalized. In this case, the oxidoreductase can activate the reaction mediator on a field free of any reaction promoter, so even the use of a high concentration reaction promoter can never induce the inactivation of the oxidoreductase. Further, the use of a high concentration reaction promoter more greatly promotes the decomposition of the hydrophobic substrate. In the seventh aspect of the invention, more preferably, the aqueous solution for the reaction is pulp solution, the hydrophilic substrate is cellulose, and the relatively hydrophobic substrate is lignin. The seventh aspect of the invention is particularly effective in such case. In this case, hydrophobic lignin as a coloring substance is sufficiently decomposed for the achievement of great bleaching, while the decomposition of cellulose as a raw paper material is suppressed, so that paper with strong folding endurance can be produced. In the seventh aspect of the invention, more preferably, the method for promoting the decomposition of the hydrophobic substrate is repeated, in multiple steps, while intermediately interposing an alkali extraction treatment of pulp. In this case, cellulose-embedded persistent lignin among lignin materials in pulp solids is exposed with the alkali extraction treatment of pulp, thereby promoting decomposition of even such lignin. Thus, the repetition of the method in multiple steps further improves the brightness of pulp. In the seventh aspect of the invention, more preferably, the oxidoreductase is a peroxidase selected from the group consisting of manganese peroxidase, horseradish peroxidase and lignin peroxidase, while the reaction mediator is manganese ion. These oxidoreductase types and reaction mediator are particularly preferable.

An eighth aspect of the invention relates to a method for decomposing a persistent chemical, which comprises allowing an oxidoreductase, a reaction mediator, and the reaction promoter in the sixth aspect of the invention to coexist in an aqueous solution containing the persistent chemical and thereby decomposing the persistent chemical with the radicalized reaction promoter. The persistent chemicals rarely are of hydrophilic structures, which is one cause of the persistency. The use of the hydrophobic reaction promoter therefor preferentially functions to shorten the decomposition time.

In the eighth aspect of the invention, more preferably, the persistent chemical is dioxin, polychlorobiphenyl (PCB) or an endocrine disruptor. These persistent chemicals are particularly important.

A ninth aspect of the invention relates to a manganese peroxidase enzyme solution obtained as or from a liquid culture of a strain SC26 (deposited as ATCC-64314) as a mutant strain of Phanerochaete crysosporium generated under ultraviolet irradiation, and the manganese peroxidase enzyme solution has the following thermal resistance (1) and hydrogen peroxide resistance (2). (1) The residual activity of manganese peroxidase after thermal treatment at 50 °C for 45 minutes is 50 % or more. (2) The residual activity of manganese peroxidase in the presence of 0.3 mM hydrogen peroxide at 37 ° C one hour later is 15 % or more. Via due procedures, the inventors of this invention obtained and cultured the Phanerochaete crysosporium strain SC26 deposited as ATCC-64314. The inventors have found that a manganese peroxidase enzyme solution with very excellent thermal resistance and hydrogen peroxide resistance (referred to as "MnP enzyme solution" hereinafter) can be obtained. Up to now, additionally, it has been found that the MnP enzyme solution contains five types of MnP isozymes, which are referred to as S1 to S5 tentatively. Because the MnP enzyme solution in the ninth aspect of the invention can exert the properties described above in (1) and (2), the enzyme solution is very useful. There is a certain quarantine regulation requiring that those who wish to obtain and import the strain SC26 should have equipment against biohazard above a given level. An individual with such equipment can receive a division thereof and can import the division. The applicant of the invention is ready to supply the MnP enzyme solution in the ninth aspect of the invention as well as the MnP contained therein to any individual for justifiable reasons.

A tenth aspect of the invention is manganese peroxidase complying with the following conditions (3) to (5). (3) The manganese peroxidase is generated by a strain SC26 (deposited as ATCC-64314) as a mutant strain of Phanerochaete crysosporium generated via ultraviolet irradiation. (4) The isoelectric point is within a range of 4.3 to 4.7. (5) The residual activity of the manganese peroxidase after thermal treatment at 50 °C for 45 minutes is 50 % or more and the residual activity of the manganese peroxidase in the presence of 0.3 mM hydrogen peroxide at 37 ° C one hour later is 15 % or more. Among the five MnP types designated as S1 to S5, the three types S1, S2 and S3 have isoelectric points within the range of 4.3 to 4.7, and all the three types have particularly great thermal resistance such that the residual activities thereof after thermal treatment at 50 °C for 45 minutes are 50 % or more. Additionally, all the three types have particularly great hydrogen peroxide resistance such that the residual activities thereof in the presence of 0.3 mM hydrogen peroxide at 37 °C one hour later are 15 % or more. From the MnP enzyme solution in the ninth aspect of the invention, a novel MnP isozyme according to the tenth aspect of the invention will possibly be found. The MnP in the tenth aspect of the invention is useful in that the MnP has excellent thermal resistance and great hydrogen peroxide resistance. Compared with the MnP enzyme solution in the ninth aspect of the invention, the MnP is practically convenient because the MnP is isolated as one single enzyme.

In the tenth aspect of the invention, more preferably, the manganese peroxidase has an isoelectric point of 4.3, and the residual activity thereof after thermal treatment at 50 °C for 45 minutes is 90 % or more and the residual activity thereof in the presence of 0.3 mM hydrogen peroxide at 37 °C one hour later is 30 % or more. In the tenth aspect of the invention, more preferably, the manganese peroxidase has an isoelectric point of 4.5, and the residual activity thereof after thermal treatment at 50 °C for 45 minutes is 90 % or more and the residual activity thereof in the presence of 0.3 mM hydrogen peroxide at 37 °C one hour later is 45 % or more. In the tenth aspect of the invention, more preferably, the manganese peroxidase has an isoelectric point of 4.7, and the residual activity thereof after thermal treatment at 50 °C for 45 minutes is 50 % or more and the residual activity thereof in the presence of 0.3 mM hydrogen peroxide at 37 °C one hour later is 15 % or more. The MnP isozymes described above are particularly highly useful.

### Mode for Carrying out the Invention

### [Modes for Carrying out the First to Fourth Aspects of the Invention]

### Reaction method

The reaction method in the first aspect of the invention is a method in which a mediator acting in an activated state thereof on a substrate to perform a predetermined reaction is charged as an active mediator in the activated state into a reaction system where the substrate exists. Herein, the term "charge" encompasses any charging of the active mediator prepared in advance by appropriate means, including charging immediately after preparation thereof or after storage by appropriate means or methods. The amount of the active mediator to be charged and the method for charging the active mediator can be determined arbitrarily, as required. Preferably, the amount can be determined on the basis of the reaction efficiency with the substrate, the amount of the substrate, the reaction time and the like. Further, the active mediator may continuously or intermittently be charged while adjusting the amount to be charged depending on the conversion of the substrate. A predetermined amount of the active mediator may be charged in each step of the reaction method.

The reaction method in the second aspect of the invention comprises an activation step of activating a mediator through the action of mediator activating means in the environment in the absence of any substrate, and a substrate reaction step of allowing the active mediator to react with the substrate after the active mediator is separated from the mediator activating means and transferred into the environment in the presence of the substrate. Preferably, when the active mediator in the second aspect of the invention is a stable low molecular substance, the reaction method comprises performing the activation step under mild conditions while putting the enzyme in contact with the mediator, and performing the reaction between the active mediator and the substrate under severe conditions at a high reaction efficiency.

The reaction method in the third aspect of the invention is a method in which the mediator in the first aspect of the invention is charged into the reaction system as a highly active mediator in a highly activated state with more improved activity and/or stability than those of the active mediator in the activated state.

### Mediator

The mediator means a so-called reaction mediating substance, which is activated into an active mediator by enzymes or other appropriate mediator activating means, for reaction with a reaction substrate to perform a predetermined reaction. Known examples include manganese ion using the enzyme manganese peroxidase as the mediator activating means, and HBT (1-hydroxybenzotriazole) and NHA (N-hydroxyacetanilide) using the enzyme laccase as the mediator activating means, all of which can be utilized in accordance with the invention. Another example is ABTS [2,2'-azino-bis-(3-ethylbenzothiazoline-6-sulphonic acid)] or veratolyl alcohol using lignin peroxidase as the activating means. Further, coenzymes such as NAD and NADP also function as the mediator of the invention in a broad sense.

### Active mediator

The active mediator means a mediator in the activated state capable of reacting with a substrate thereby performing a predetermined reaction. The reaction of the mediator activating means to be described below with a mediator expresses the active mediator. The active mediators include general active mediators and highly active mediators. General active mediators mean active mediators in general activated states and in free states, like trivalent manganese ion. Highly active mediators mean mediators in highly activated states particularly with more improved activities and/or stabilities than those of general active mediators, including for example active mediators in a more highly active and excited state than general active mediators, or general active mediators modified into a form of high-order compound including at least complex to acquire higher stability. Examples of the active mediator in a particularly highly active and excited state include metal complex with high redox potential, mixtures of oxidizing reagents (metal ion and the like) with diketone and/or derivatives thereof, complexes of metals with diketone and/or derivatives thereof, radicals of ketones (diketone and derivatives thereof, and the like). Examples of the highly active mediator with particularly improved stability include active mediators stabilized via bonding with stabilizers (for example, complexes of trivalent manganese ion with malonic acid or diketone structures or the like).

### Mediator activating means

In the reaction method in accordance with the first or the third aspect of the invention, the mediator activating means includes, but is not limited to, enzyme. Detailed description will be made about enzyme, below, but mediator activating means except for enzyme may be selected arbitrarily, depending on the type of an active mediator or the aim of the use thereof. For example, mediators can be activated with catalysts except for enzyme, or can be activated by photoirradiation, electromagnetic irradiation, voltage application or by plasma preparation of the mediators. As the catalysts, various chemical substances with general catalytic actions, photocatalysts and the like can be utilized. The chemical substances are not limited as far as they are electron donor substances. Examples of the chemical substances include metal ions, metal complexes, organic acids, aromatic carboxylic acids and the like. The photoirradiation may be utilized effectively for photocatalysts, the example of which is titanium oxide. Examples of mediator activation by electromagnetic irradiation, voltage application or plasma preparation include substances with a deviation in electron charge, such as electron transfer complexes, and substances in the excitation state.

In accordance with the reaction method in the second aspect of the invention, the mediator activating means is an enzyme in which the mediator is involved. As far as the enzyme has an enzymatic reaction mechanism in which a mediator is involved, the type of the enzyme is not limited. Typical examples include oxidoreductases such as manganese peroxidase and laccase.

In the case that an activation reaction field is separated from a substrate reaction field through a semi-permeation membrane with no permeability of any enzyme, the enzyme may be charged in a state of a simple aqueous solution into the activation reaction field. Generally, however, the enzyme is preferably immobilized with an insoluble carrier. As to modes of the immobilization and the use thereof, an enzyme immobilized on an insoluble carrier in powder or granule may be dispersed into a mediator solution; or an immobilized enzyme may be filled in a column through which a mediator solution passes; or an enzyme may be immobilized on a carrier as a material composing an immobilization bed through which a mediator solution passes. One preferable example of the insoluble carrier is a porous carrier; one preferable example of the porous insoluble carrier is a mesoporous material with numerous mesopores of dimensions corresponding to enzymes of general sizes, where enzymes immobilized in these mesopores can be insolubilized and stabilized. Particularly, mesoporous silica materials with pores (referred to as 'FSM' hereinafter) are exemplified, which are prepared from a raw material of lamellar silicate for example Kanemite, utilizing a template substance for pore formation, and the like.

In the case that the mediator can be activated by means other than enzymes on the activation field, such means may also be used. In the case that manganese ion as the mediator for manganese peroxidase is activated into trivalent ion, for example, such means include oxidizing manganese oxide with an appropriate chemical means, and reducing manganese dioxide. Further, these oxidation and reduction may also be carried out electrically as described above. In this case, it is preferable that a chelating agent is contained in a mediator solution to stabilize trivalent manganese ion.

### Substrate

Once the mediator and the enzyme (or mediator activating means except for enzyme) have been determined, the substrate type or category can be determined automatically. Taking oxidoreductases such as manganese peroxidase and laccase as examples, however, the category of substrates and the type of industrial materials to be treated including substrates range very diversely. One example of the most preferable combination of a substrate and industrial material to be treated is pulp as the material to be treated and lignin in pulp to be decomposed for pulp bleaching as the substrate.

### Reaction apparatus

The reaction method in the second aspect of the invention is carried out, preferably, by using a reaction apparatus in the fourth aspect of the invention. The activation step and the substrate reaction step are carried out on the activation reaction field and on the substrate reaction field in the fourth aspect of the invention, respectively. It is at least required that the activation reaction field and the substrate reaction field are retained in a separated state from each other to prevent the transfer of the substrate from the latter to the former, and the fields are in communication with each other through a transfer means capable of transferring the active mediator from the former to the latter. Provided that these conditions are satisfied, the reaction fields may be formed, for example, as reaction containers or reaction tanks in appropriate forms and independent of each other, or may be composed of separated fractions in one reaction container or one reaction tank with a separation membrane or a separation wall. The transfer means is not limited. For examples, a pipe for liquid transfer (preferably equipped with an enforceable transfer capacity with a pump or the like) is preferably used, because mediators are generally in the state of aqueous solution and so the active mediators are transferred in aqueous solution. If necessary, the transfer means may be equipped with separation means (for example, solid-liquid separation means such as filter) for separating mediator activating means from an active mediator. It is only required for the activation reaction field to be equipped with mediator activating means in a separable form from at least the active mediator. The 'separable form' is not limited, but a fixed structure or insoluble solid is preferable when the active mediator is for example in the form of aqueous solution. As the fixed structure, electrode is exemplified when the mediator is a metal ion activated via the change of the valence. As the insoluble solid, enzymes immobilized on insoluble carriers are exemplified.

On the activation reaction field, a mediator is provided in the form of aqueous solution and the like, to activate the mediator by mediator activating means. Then, the active mediator is transferred onto the substrate reaction field. A series of these processes can be continuously and successively performed. No substrate is provided onto the activation reaction field. Water functioning as the medium for the mediator and the active mediator may be prepared as buffer solution, taking account of the stabilization of the reaction conditions. Further, a stabilizer for stabilizing the active mediator may be contained in the resulting buffer solution, if necessary. For example, trivalent manganese ion as an active mediator for the enzyme reaction with manganese peroxidase loses its activity when the trivalent manganese ion is modified into divalence. It is still known that in the presence of prescribed chelating agents such as malonic acid, nevertheless, the ion is bound to the complex so that the resulting trivalent manganese ion is stabilized. A substrate is fed on the substrate reaction field, while from the activation reaction field, an active mediator is fed through the transfer means. The reaction apparatus may be so structured that the substrate and the active mediator are continuously and successively fed, and the substrate after the predetermined treatment on the substrate reaction field is successively transferred to the subsequent process.

Reaction conditions such as pH and temperature on the activation reaction field and the substrate reaction field may be set by appropriate means. For example, the reaction conditions may be set by pH adjustment of the mediator solution fed onto the activation reaction field, addition of pH adjuster on the substrate reaction field and temperature adjustment on both of the reaction fields, and the like. Specific reaction conditions may arbitrarily be determined, as required. In the case that the mediator activating means is an enzyme and the resulting active mediator is a stable low molecular substance, as in the second aspect of the invention, preferably, the activation step is performed under mild conditions to avoid enzyme inactivation and the substrate reaction step is performed under severe conditions at a high reaction efficiency to raise the efficiency of the treatment of the substrate. In this case, the active mediator as a stable low molecular substance is not inactivated. Even when a concern exists for the stability of the active mediator, the loss of the stability is not caused by the inactivation mechanism due to the change of the steric structure as in enzyme. So the stabilization thereof by stabilization treatment, as in the stabilization of trivalent manganese ion with the chelating agent, can be made by various methods.

### [Mode for Carrying out the Fifth Aspect of the Invention]

### Oxidoreductase and oxidizing reagent

The oxidoreductase is an oxidoreductase of a type decomposing a substrate utilizing an oxidizing reagent. Such oxidoreductase is generally referred to as peroxidase, but as far as the peroxidase is an oxidoreductase of a type utilizing an oxidizing reagent, the peroxidase is encompassed within the oxidoreductase in the fifth aspect of the invention, despite the name thereof or the oxidizing reagent type. Typical examples of such oxidoreductase include manganese peroxidase, lignin peroxidase, laccase and the like. As the oxidizing reagent, typical one is hydrogen peroxide. Besides, oxygen and organic peroxides such as peracetic acid are exemplified.

The oxidizing reagent is used within a concentration range allowed by an immobilized enzyme. The concentration range varies, depending on the types of the oxidoreductase, oxidizing reagent and persistent chemical and the reaction conditions of temperature, pH and the like, so the concentration range is not definitely defined. However, one example is as follows: manganese peroxidase immobilized in the conventional manner as mentioned above is inactivated rapidly with hydrogen peroxide at a concentration of about 0.5 to 1 mM, while the present immobilized enzyme using the same manganese peroxidase is never inactivated for a considerable period of time with hydrogen peroxide generally at a concentration of 6 mM, about 6-fold to 12-fold the concentration. Conventionally, for examples, for bleaching pulp using manganese peroxidase, it has been required for enzymes such as glucose oxidase to generate hydrogen peroxide under controls, because the sensitivity of the hydrogen peroxide sensor is above about 0.1 mM. However, the use of such immobilized enzyme enables control of hydrogen peroxide at a high concentration, so that the immobilized enzyme has a great advantage in that the control can be done with inexpensive aqueous hydrogen peroxide.

### Immobilized enzyme

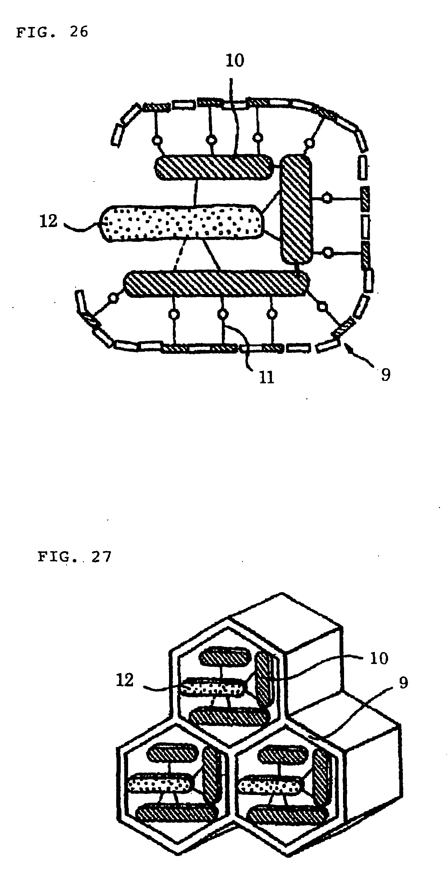

Oxidoreductase is immobilized in a structure unit with structure stability and with a dimension approximately fitting to the enzyme size. The entirety of the enzyme protein molecule may be immobilized in the structure unit, or the enzyme active unit (enzyme fragment including the active site) may be immobilized. As shown in Figs. 26 and 27, structure unit 9 is in a hollow structure with structure stability against environmental conditions such as pH, heat and fluidity of fluids, and in the inside thereof, enzyme 10 (the enzyme active unit may be used) is immobilized. The inner diameter of the structure unit 9 is preferably in a dimension approximately fitting to the enzyme size of the enzyme 10.

Anchor unit 11 is not an essential structure element for the immobilized enzyme, but an element connecting the structure unit 9 and the enzyme 10 together. It works to transfer the structure stability of the structure unit 9 to the enzyme 10 to suppress the inactivation of the enzyme 10 due to severe modification of the steric structure, and to give freedom at a level allowing a relatively small structure modification of the active site required for the interaction with substrate 12. The molecule composing the anchor unit is preferably of essentially the same structure as that of the structure unit, and for the connection to the enzyme, for example, functional groups such as hydroxyl group, amino group, pyridine group, urea group, carboxylic acid group and hydroxyl group should be bound to the anchor unit molecule. The oxidoreductase immobilized in the structure unit may be connected with the structure unit not through the anchor unit but through van der Waals force. One or a small number of enzyme molecules are placed in each of the structure units.

The structure unit is made of inorganic materials or organic materials such as polymer, and the material type is not specifically limited as far as the material type does not impede the purposes of the invention. The structure unit of the inorganic material may be made of various oxides of metals such as silicic acid and alumina, and complex oxides of metals such as Si and Al and the like. For a method for forming a structure unit containing silicic acid, for example, lamellar silicates such as Kanemite, silica gel, water glass and sodium silicate can preferably be used. Particularly preferable is a mesoporous silica material in the form of an assembly of a great number of structure units containing silicic acid. The method for preparing the mesoporous silica material is not limited, but a preferable example of the method comprises: mixing a lamellar silicate for example Kanemite with a template substance surfactant (cation surfactants such as alkyltrimethylammonium, anion surfactants such as alkylsulfonate salt, and nonionic surfactants such as polyethylene glycol are usable) for reaction, to form a surfactant/inorganic complex where an inorganic backbone is formed around the micelle of the surfactant, and removing the surfactant for example by sintering at 400 to 600 °C or by organic solvent extraction or the like, to form a mesopore of the same shape as the shape of the micelle of the surfactant in the inorganic backbone.

In the case that the structure unit is formed using a lamellar silicate such as Kanemite, the surface of the pore turns hydrophobic and has anionic properties. The hydrophobic surface is favourable for stable non-hydrated enzyme immobilization, while the anionic surface is favorable for the immobilization of an enzyme having cations such as amino group on the surface.

As described above, the structure unit is preferably in a dimension approximately fitting to the enzyme size. The dimension (namely, pore diameter) of such structure unit in the mesoporous silica material can be adjusted by modifying the length of the alkyl chain in the surfactant to adjust the micelle diameter. By using relatively hydrophobic molecules such as trimethylbenzene and tripropylbenzene in combination with the surfactant, the micelle can be swelled to consequently form a larger structure unit. The pore diameter is generally 1 to 30 nm, preferably 2 to 10 nm. The immobilized enzyme thus prepared is in the form of powder, granule, sheet, bulk, membrane or the like. This is used through dispersion and charging into a material to be treated, filling in a column through which a solution to be treated passes, or construction of an immobilization bed through which a solution to be treated passes, or in the form of film or multi-layer assembly, or the like.

### Substrate

The type of a substrate to be treated is not limited, as far as the substrate can finally be decomposed with peroxidase as an oxidoreductase, using oxidizing reagents. For example, phenols such as guaiacol and pyrogallol may arbitrarily be used as the substrate. Particularly preferable is a case that the substrates are persistent chemicals of which decomposition treatment is socially desired. Several examples of such persistent chemicals include lignin as a coloring substance to be decomposed in pulp bleaching, and dioxin, bisphenol A and polychlorobiphenyl (PCB) and the like, which are problematic as endocrine disruptors.

### [Mode for Carrying out the Sixth to Eighth Aspects of the Invention]

### Reaction promoter

The reaction promoter is used in a substrate decomposition reaction system with oxidoreductases and essentially satisfies the following two conditions: the promoter has a diketone structure shown below as Chemical formula 1; and is soluble in water. The reaction promoter can procure constant hydrophobicity due to the diketone structure.

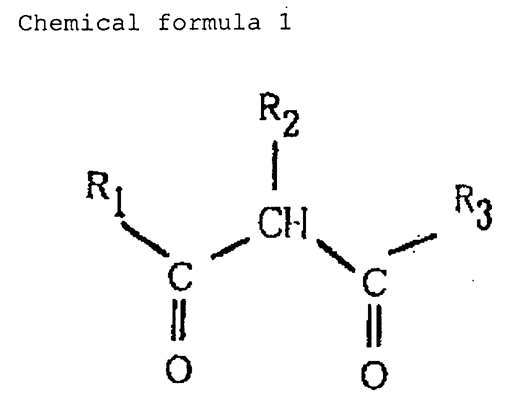

In the Chemical formula 1, R1, R2 and R3 are not limited, provided at least that R1, R2 and R3 are not hydroxyl group or alkoxyl group. Specifically, R1, R2 and R3 are appropriately selected from: hydroxyl group; alkyl groups with low molecular weights, such as methyl group, ethyl group and isopropyl group; derivative groups of those alkyl groups (for example, dibromomethyl group and trifluoromethyl group); 5-membered carbon rings or 6-membered carbon rings such as phenyl group, 5-membered or 6-membered heterocyclic rings such as thiophen group. R1, R2 and R3 may be the same groups or may be different groups from each other. However, it should be avoided that the selection of R1, R2 and R3 groups results in such a high molecular weight that the resulting reaction promoter turns insoluble in water. A particularly preferable reaction promoter is acetylacetoaldehyde where R2 is hydrogen and either one of R1 and R3 is hydrogen while the other is methyl group, or acetylacetone where both R1 and R3 are methyl group. Acetylacetone is particularly preferable.

### Oxidoreductase

Any oxidoreductase capable of activating a reaction mediator thereby radicalizing the reaction promoter can be used with no limitation. Oxidoreductase is preferable; and peroxidase is particularly preferable. The type of peroxidase is not limited, but manganese peroxidase, horseradish peroxidase or lignin peroxidase can preferably be used. More preferably, the oxidoreductase is immobilized with an insoluble carrier. The immobilization and the use thereof is preferably such that the enzyme is immobilized on or in an insoluble carrier in powder or in granule for use. One preferable example of the insoluble carrier is a porous carrier. One preferable example of the porous insoluble carrier is a mesoporous material with numerous mesopores in a dimension corresponding to an enzyme of a general size, where the enzyme is placed in these mesopores for insolubilization and stabilization, and particularly mesoporous silica materials formed from a raw material of lamellar silicate such as Kanemite, utilizing a template substance for pore formation and the like.

### Reaction mediator

The reaction mediator means a mediator activated with a specific enzyme into an active mediator, to radicalize a reaction promoter. The reaction mediator is sometimes determined depending on the type of the oxidoreductase. For example, manganese ion is utilized for manganese peroxidase, while veratolyl alcohol is utilized for lignin peroxidase. A mediator is particularly preferably a stable low molecular substance, of which the active form can retain the activity under severe conditions at high reaction efficiency. Even when the active form is not completely stable under the conditions, the reaction mediator is also preferable if the mediator can be stabilized by a given treatment, for example, by binding to stabilizers.

### Method for promoting decomposition of hydrophobic substrate

A first method for promoting the decomposition of a hydrophobic substrate comprises allowing an oxidoreductase, a reaction mediator and a reaction promoter to coexixt in an aqueous solution where a hydrophilic substrate and a substrate hydrophobic relatively to the hydrophilic substrate exist in mixture, to radicalize the reaction promoter to thereby preferentially decompose the hydrophobic substrate. A second method for promoting the decomposition of a hydrophobic substrate comprises activating a reaction mediator with an oxidoreductase, and adding the activated reaction mediator and a reaction promoter to an aqueous solution where a hydrophilic substrate and a substrate hydrophobic relatively to the hydrophilic substrate exist in mixture, to radicalize the reaction promoter to thereby preferentially decompose the hydrophobic substrate.

For these methods, the types of the oxidoreductase and the reaction mediator are as described above. The substrate solution to be treated in accordance with the methods is a substrate solution in water as a medium, where a hydrophilic substrate and a substrate hydrophobic relatively to the hydrophilic substrate coexixt in mixture. The types of the hydrophilic substrate and the hydrophobic substrate are not limited. A particularly preferable substrate solution is a pulp solution to be treated for bleaching. Namely, the substrate solution is such that the hydrophilic substrate is cellulose of which the decomposition with oxidoreductase should be avoided essentially, while the substrate hydrophobic relatively thereto is a coloring substance lignin to be decomposed. When the substrate solution is a pulp solution to be treated for bleaching, it is preferable for more improving the bleaching level of pulp, to repeat the method for promoting the decomposition of a hydrophobic substrate, in multiple steps, while intermediately performing an alkali extraction treatment of pulp.

### Method for decomposing persistent chemicals

The method for decomposing a persistent chemical comprises allowing an oxidoreductase, a reaction mediator and a reaction promoter to coexist in an aqueous solution containing the persistent chemical to decompose the persistent chemical with the radicalized reaction promoter. The type of the persistent chemical is not limited, but dioxin, PCB or an endocrine disruptor is particularly important. The endocrine disruptor includes for example bisphenol and nonylphenol.

### [Mode for Carrying out the Ninth to Eleventh Aspects of the Invention]

### MnP enzyme solution

The MnP enzyme solution in the ninth aspect of the invention can be obtained as a liquid culture of the strain SC21 as a Phanerochaete crysosporium mutant strain obtained via ultraviolet irradiation. Otherwise, the liquid culture is filtered or centrifuged to remove the solids therein, to thereby obtain an MnP enzyme solution at a higher purity. Further, by salting-out or column chromatography or the like, an MnP enzyme solution at an even higher purity can be obtained. The MnP enzyme solution in accordance with the ninth aspect of the invention is at a residual MnP activity of 50 % or more after thermal treatment at 50 °C for 45 minutes, and is at a residual MnP activity of 15 % or more in the presence of 0.3 mM hydrogen peroxide at 37 °C one hour later. No MnP enzyme solution with such high-level thermal resistance and hydrogen peroxide resistance has been known yet. Because the enzyme solution contains at least five types of MnP isozymes, as referred to as S1 to S5 by the present inventors, characteristics of the enzyme solution may reflect the characteristics of all the MnP isozymes. The MnP enzyme solution in the ninth aspect of the invention may satisfactorily be supplied as the enzyme solution with various purities for commercial distribution or utilization. Otherwise, the enzyme solution may satisfactorily be prepared into various dosage formulations, such as frozen formulation or freeze-dried formulation, by various processing means for enzyme formulations. The resulting formulations may then be supplied for commercial distribution or utilization.

### MnP

The MnP in the tenth aspect of the invention is generated by the strain SC21 as the mutant strain of Phanerochaete crysosporium. The MnP has an isoelectric point within a range of 4.3 to 4.7, and a residual activity of 50 % or more after thermal treatment at 50 °C for 45 minutes and a residual activity of 15 % or more in the presence of 0.3 mM hydrogen peroxide at 37 °C one hour later. Among the MnP types, particularly preferable are the MnP isozymes designated as S1, S2 and S3. None of these MnP isozymes have been reported or obtained yet.

S1 has an isoelectric point of 4.3, and a residual activity of 90 % or more after thermal treatment at 50 °C for 45 minutes and a residual activity of 30 % or more in the presence of 0.3 mM hydrogen peroxide at 37 °C one hour later. The molecular weight of S1 is 40.5 kDa.

S2 has an isoelectric point of 4.5, and a residual activity of 90 % or more after thermal treatment at 50 °C for 45 minutes and a residual activity of 45 % or more in the presence of 0.3 mM hydrogen peroxide at 37 °C one hour later. The molecular weight of S2 is 40.5 kDa.

S3 has an isoelectric point of 4.7, and a residual activity of 50 % or more after thermal treatment at 50 °C for 45 minutes and a residual activity of 15 % or more in the presence of 0.3 mM hydrogen peroxide at 37 °C one hour later. The molecular weight of S3 is 40.5 kDa.

### Method for producing MnP

The method for preparing MnP enables the production of at least one or more kinds of the MnP enzyme solutions in the ninth aspect of the invention or the MnP in the tenth aspect of the invention. More specifically, the SC21 strain is cultured, from which any of the MnP enzyme solutions or the MnP can be collected. The culture conditions of the strain SC21 are not limited. The culture medium type and additives and the like are not specifically limited. The culture method is not specifically limited, either, but preferably, the strain can be cultured, for example, by placing one liter of an appropriate culture medium in a 2-liter Erlenmeyer flask, adding 80 ml of a bacterial suspension obtained by homogenizing pre-culture bacteria after preliminary culture for 2 days and then culturing the bacteria under shaking at 30 °C and 120 rpm for an appropriate period of time. As to the culture conditions except for those described above, for example, enzyme inducers such as veratolyl alcohol are preferably added on the third day of the culture, or oxygen is preferably filled in the culture flask followed by tight sealing. The substitution with oxygen is preferably performed once a day from the third day of the culture until the termination of the culture.

So as to improve the purity of the resulting MnP enzyme solution in obtaining the MnP enzyme solution by the preparation method, various procedures, such as the filtration and centrifugation of the liquid culture and the salting-out and column chromatography for higher purity, can be carried out, as described above. From the culture of the strain SC21 or from the MnP enzyme solutions, MnP can be obtained and purified, individually. The obtaining and purifying processes are not limited, but procedures such as anion exchange chromatography and cation exchange chromatography may arbitrarily be carried out.

### MnP immobilization

For the use of various types of MnP in the tenth aspect of the invention, MnP is more preferably immobilized with an insoluble carrier so as to more improve the thermal resistance and hydrogen peroxide resistance. For the immobilization and use, the enzyme may be immobilized on or in an insoluble carrier in powder or granule, which may then be dispersed and charged into a mediator solution. Otherwise, the immobilized enzyme may be filled in a column through which the mediator solution is allowed to pass. Or the enzyme may be immobilized on a carrier as a material composing an immobilization bed through which the mediator solution passes. One preferable example of the insoluble carrier is a porous carrier. One preferable example of the porous insoluble carrier is a mesoporous material having numerous mesopores in a dimension corresponding to general enzyme sizes, where an enzyme is placed in these mesopores to insolubilize and stabilize the enzyme, and particularly a mesoporous silica material (FSM) prepared from a raw material of lamellar silicate for example Kanemite, utilizing a template substance for pore formation and the like. MnP utilization

The MnP enzyme solutions or various MnP can be utilized for various uses. For example, they can be used for the decomposition of lignin in paper pulp for paper pulp bleaching and the decomposition of persistent chemicals hazardous for humans and the like. The hazardous persistent chemicals include various endocrine disruptors, such as dioxin, PCB, bisphenol and nonylphenol. In addition to those described above, efficient decomposition of chemical fibers such as nylon and other plastics and the like are exemplified as preferable uses of the various MnP enzyme solutions or various MnP. For enzyme reaction with the MnP enzyme solutions or MnP, manganese ion as a mediator and hydrogen peroxide are allowed to coexist therein.

The mediator means a reaction-generated substance via enzyme reaction, which is activated into an active mediator (divalent manganese → trivalent manganese) via the action of the enzyme MnP and then reacts with the substrate to perform a predetermined reaction. The trivalent manganese ion as an active mediator is allowed to coexist with prescribed chelating agents such as malonic acid, thereby binding the manganese ion to the chelating agents to thereby stabilize the manganese ion in its trivalent state. Water as the medium for the mediator and the active mediator may be prepared as a buffer solution, taking account of the stabilization of the reaction conditions.

Hydrogen peroxide concentration gives significant influence on the rate of the enzyme reaction with the MnP enzyme solution or MnP. In the enzyme reaction utilizing the conventional manganese peroxidase, the substantial upper limit of the hydrogen peroxide concentration was about 0.1 mM, to avoid the inactivation of the enzyme. In the present invention using the MnP enzyme solution or MnP, the hydrogen peroxide concentration can be raised up to about 0.3 mM (one hour) . In the case that the MnP in the tenth aspect of the invention is used as the FSM-MnP, the hydrogen peroxide concentration can be further raised up to about 6 mM (one hour).

For the enzyme reaction of the MnP enzyme solution or MnP in accordance with the invention, the reaction temperature gives significant influence on the rate of the enzyme reaction, as generally remarked for enzyme reactions. So as to avoid enzyme inactivation during enzyme reactions utilizing manganese peroxidase in the prior art, the substantial upper limit of the reaction temperature was about 30 °C. For the MnP enzyme solution or MnP in accordance with the invention, the reaction temperature can be raised up to about 50 °C over one hour or more. In the case that the MnP of the invention is used as the FSM-MnP, the reaction temperature can be further raised up to about 60 °C over one hour or more.

### Examples

### [Examples of the First to Third Aspects of the Invention]

### (Example 1 - Construction example of small-scale reaction apparatus)

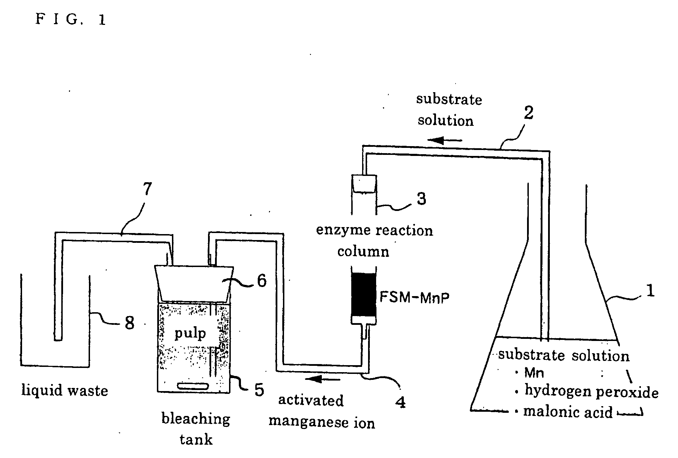

One example of a reaction apparatus of a simple construction is shown in Fig. 1. Container 1 contains an aqueous mediator solution. The aqueous mediator solution may satisfactorily be an appropriate buffer solution and may contain stabilizers such as chelating agents for stabilizing active mediator. The inside of the container 1 is in communication through glass tube 2 with the top end of reaction column 3 as an activation reaction field. The inside of the reaction column 3 is filled with a mesoporous silica material, in granule, where an enzyme is placed and immobilized in the numerous mesopores therein. The enzyme performs an enzyme reaction mediated by the mediator. The lower end of the reaction column 3 is in communication through glass tube 4 with the inside of container 5 as a substrate reaction field. The substrate (insoluble solid matter) for the enzyme is contained in fragments in a state of aqueous suspension in the container 5. The container 5 is sealed tightly with stopper 6 to form a closed system like the reaction column 3. The inside of the container 5 is in communication through glass tube 7 with the inside of container 8 as a liquid waste reservoir. Further, a pump (not shown) is arranged on any of the glass tubes 2, 4 and 7, and the aqueous solution in the container 1 is enforced to move at a predetermined flow volume as designed, sequentially to the reaction column 3, the container 5 and the container 8.

With this construction, the mediator in the aqueous solution placed in the container 1 moves into the reaction column 3 as the activation reaction field through the glass tube 2, where the mediator is activated by the enzyme. Then, the active mediator is transferred into the container 5 as the substrate reaction field to trigger a predetermined reaction toward a substrate, and an aqueous solution of the mediator inactivated through the reaction is disposed into the container 8. In this Example, if the substrate is a water-soluble substance, the container 8 may be used as a reaction container for effecting the subsequent step and thus the substrate after the predetermined reaction in the container 5 may be transferred, together with the mediator after use, into the container 8.

### (Example 2 - Separation between activation step and substrate reaction step)

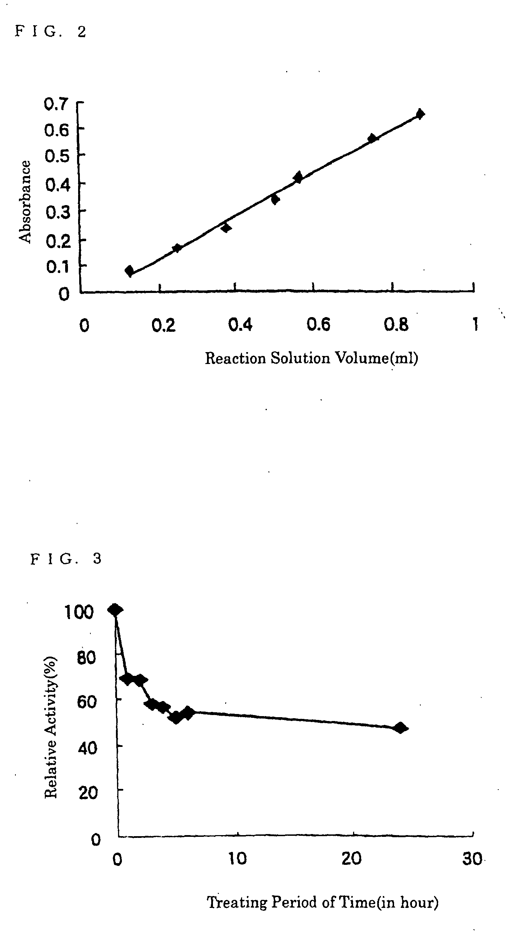

Five mg of a powder of FSM with immobilized manganese peroxidase of given activity units (manganese peroxidase is referred to as 'MnP' hereinafter) immobilized therein (FSM with immobilized MnP is referred to as 'FSM-MnP' hereinafter) was suspended in a buffer solution [25 mM succinate buffer (pH 4.5), 1 mM sodium malonate, 0.5 mM manganese sulfate, 0.1 mM hydrogen peroxide] containing divalent manganese as a mediator, and malonate salt as a stabilizer of the active mediator (trivalent manganese) for reaction at 37 °C for 5 minutes, and the resulting reaction mixture was centrifuged to remove the immobilized enzyme to obtain the reaction supernatant. It was confirmed by an enzyme titer assay that the reaction supernatant contained trivalent manganese with no MnP. The reaction supernatant was mixed with a given volume of an aqueous solution containing a given volume of 2,6-dimethoxyphenol (DMP) of a predetermined excessive concentration as the substrate, and the resulting developed color was measured at the absorbance at 470 nm to assay the DMP oxidation potency due to the reaction supernatant. The results are shown in the graph of Fig. 2. The "reaction solution volume (ml)" on the abscissa in Fig. 2 represents the volume of the reaction supernatant mixed with the given volume of the aqueous solution of the substrate. In a separate comparative test, the same procedures as described above were carried out using FSM with no MnP immobilized, and it was confirmed that no color development of DMP was observed when its reaction supernatant was used.

As apparently shown in Fig. 2, the reaction supernatant with no content of MnP in the present Example allowed DMP to develop color, and the intensity of the developed color was increased corresponding to the volume of the reaction supernatant. Based on this, it was confirmed that the activation step and the substrate reaction step were separable from each other as in the first aspect of the invention for such mediator-mediated enzyme reaction.

### (Example 3- Stability of active mediator)

The reaction supernatant obtained in Example 2 was sealed in a container and left to stand in ice, to assay the residual quantity of trivalent manganese in the reaction supernatant, using as the marker the oxidation activity of DMP after given periods of time passed. The results are shown in relative activity taking the oxidation activity of DMP immediately after the reaction supernatant in Example 2 was obtained as 100 %, and are depicted in the graph of Fig. 3. The "treating period of time (in hour)" on the abscissa in Fig. 3 means the period of time in hour when the supernatant was left to stand in ice. According to the results, trivalent manganese ion as the active mediator in the reaction supernatant remained at 60 %, 6 hours later and about 40 %, 24 hours later. This shows that when at least trivalent manganese is stabilized with the chelating agent (malonic acid), the active mediator is sufficiently stable over the period of time generally required for the active mediator to move from the activation reaction field to the substrate reaction field.

### (Example 4- Enzyme stability)

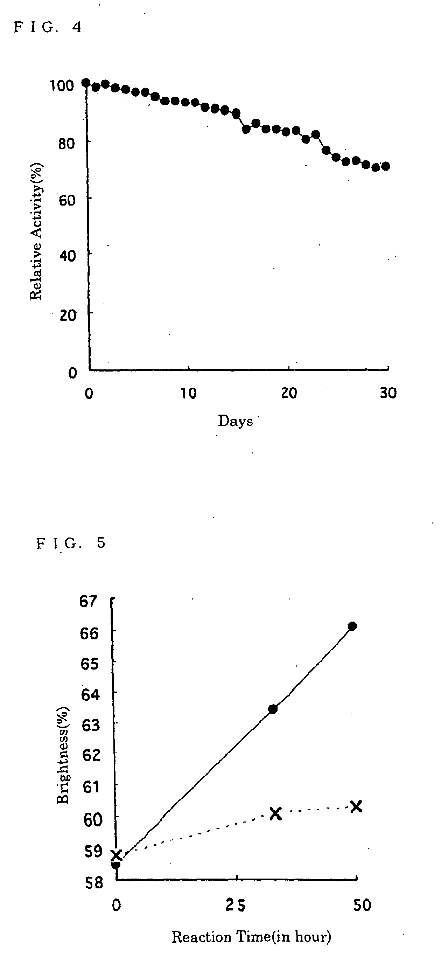

Slightly coarse granules of FSM in which MnP of predetermined activity units was immobilized were filled in a column, through which a buffer solution [50 mM malonate buffer, pH4.5, 0.1 mM manganese sulfate, 0.05 % Tween 80, 0.1 mM hydrogen peroxide] at 39 °C was allowed to flow down at a flow rate of 200 mL/hr. for one hour, to continuously generate trivalent manganese. After the termination of the aforementioned procedures, the porous material in granule was rinsed with distilled water and stored at 4 °C. The same procedures were repeated on the same porous material in granule once a day for 30 days. Then, the stability of the enzyme activity of MnP immobilized on the porous material in granule was evaluated, using as the marker the oxidation activity of DMP due to the flow-through solution from the column. The results are shown in the graph of Fig. 4, as represented in relative activity taking the DMP oxidation activity due to the flow-through solution at the procedure on the first day as 100 %.

According to the results, the DMP oxidation activity due to the flow-through solution was gradually decreased as the days passed, but even after 30 days, the flow-through solution retained about 70 % relative activity. The results indicate the stability of the enzyme immobilized in FSM in one aspect but also indicate that the involvement of the enzyme in the activation step alone, spatially separately from the substrate reaction step, is advantageous for the enzyme stability in such mediator-involved enzyme reaction.

### (Example 5- Pulp bleaching)

Under the same conditions, the same buffer solution as in Example 4 was allowed to flow through a column prepared in the same manner as in Example 4, and then, the flow-through solution was continuously fed into a reaction tank (substrate reaction field) placing therein 1 % unbleached pulp, to thereby effect pulp bleaching. These procedures were all carried out at 39 °C. In a comparative example, alternatively, the same buffer solution was allowed to flow through a column packed with FSM in coarse granule but without any enzyme immobilization. Using the flow-through solution, the same pulp bleaching test was carried out.

These results are shown in the graph of Fig. 5 depicting the change of pulp brightness (%) vs. the reaction time after the start of the bleaching test, where the brightness of pulp is effectively improved in the present Example plotted with black dots, with significant difference from the comparative example plotted with the symbol "×".

### (Example 6- Trivalent manganese reactivity at each temperature)

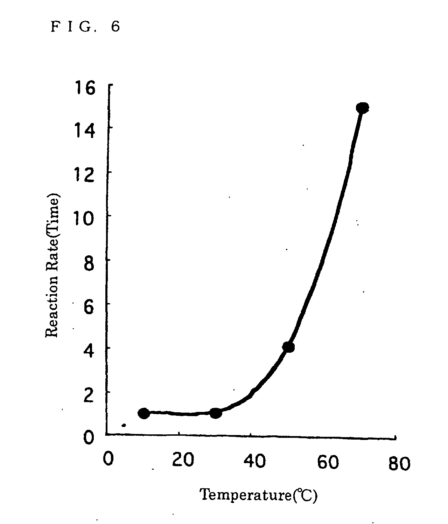

One milliliter of a substrate solution [30 mM malonate buffer solution (pH 4.5), 10 mM manganese sulfate, 0.1 mM hydrogen peroxide] was added to 50 µl of FSM with immobilized MnP of given activity units, for reaction at 37 °C for 5 minutes. The immobilized enzyme was separated via centrifugation, and the resulting reaction supernatant was stored as activated Mn solution in ice. The activated Mn solution was kept warm at. each temperature for 10 minutes, to assay the quantity of eliminated Mn (III), namely eliminated trivalent manganese, using as the marker the absorbance change at 270 nm. Fig. 6 shows the quantity of eliminated Mn (III), namely the reaction rate at each temperature. Mn (III) forming a complex with malonic acid was retained stably with almost no reaction (reduction reaction) in a low temperature zone below about 30 °C, but in a high temperature zone above about 40 °C, the reaction rate was rapidly increased as the temperature was raised. This indicates that the contact of Mn (III) with pulp at high temperature efficiently enables the reaction with pulp.

### (Example 7- Relation of the reaction temperatures of enzyme column and reaction tank with pulp bleaching efficiency)

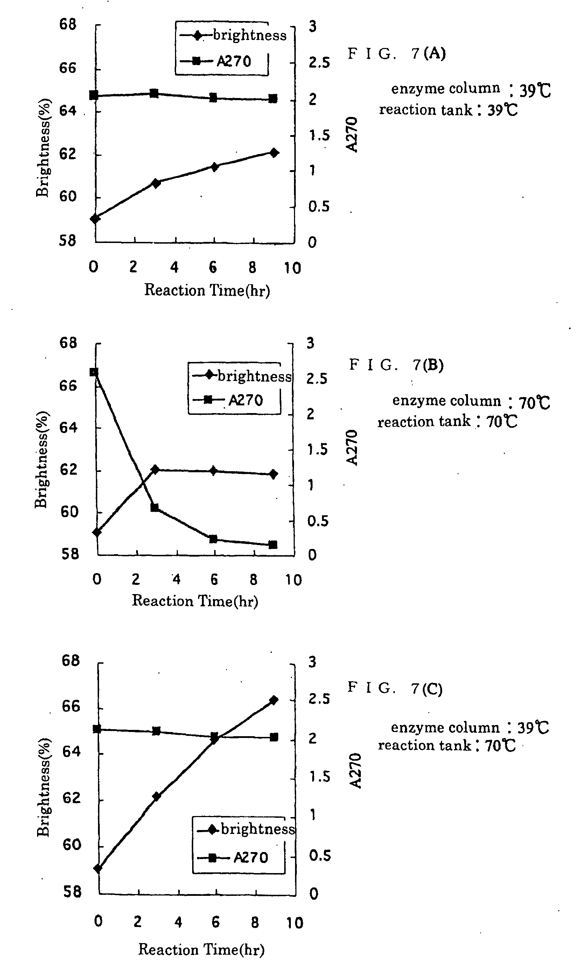

0.4 mL of FSM with immobilized MnP of given activity units was filled in a column, through which an activated buffer [10 mM manganese sulfate, 0.05 % Tween 80, 0.1 mL hydrogen peroxide, 30 mM malonate buffer (pH 4.5)] was allowed to flow down at a flow rate of 360 mL/hr. to continuously generate Mn (III). By continuously feeding the enzyme reaction solution containing Mn (III) into a reaction tank placing therein 1 % unbleached pulp, the pulp was bleached. During this procedure, the enzyme column and the reaction tank were kept warm at 39 °C or 70 °C. When the enzyme column and the reaction tank were both kept at 39 °C (Fig. 7 (A)), the enzyme activity was stably maintained over about 10 hours, but the pulp bleaching efficiency was low. When the enzyme column and the reaction tank were both kept at 70 °C (Fig. 7(B)), the enzyme activity was rapidly decreased immediately after the start of the reaction, while the increase of the brightness was rapid at the early stage. However, the increase of the brightness was no more gained as the enzyme was inactivated. When the enzyme column and the reaction tank were kept at 39 °C and 70 °C, respectively (Fig. 7(C)), the enzyme activity was stably maintained, while the bleaching rate was rapid, indicating that pulp was most efficiently bleached then. Based on the results in Figs. 7(A) to 7(C), it was confirmed that more efficient enzyme reaction could be realized by individually setting the temperature of the enzyme column and the temperature of the reaction tank to the respective optimal temperatures.

### (Example 8- Multi-step bleaching with combination of enzyme treatment/alkali extraction)

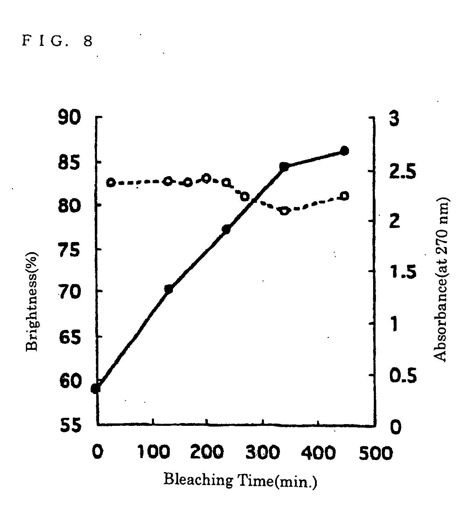

Unbleached pulp was bleached by alternately repeating the enzymatic bleaching with MnP-immobilized FSM and alkali extraction. In the system shown above in Example 7, 60-minute enzymatic bleaching was done by keeping the enzyme column and the reaction tank at 39 °C and 70 °C, respectively. Subsequently, the pulp was suspended in an alkali solution (2.5 % sodium hydroxide) and kept warm at 70 °C for 5 minutes. After rinsing the pulp, the procedures were repeated until the intended brightness (brightness of 85 %) was yielded. Consequently, seven cycles of the enzymatic bleaching and seven cycles of the alkali extraction were repeatedly carried out, until the intended brightness was yielded in total of about 450 minutes, as shown in the graph plotted with black dots in Fig. 8. It was confirmed that the brightness reached an industrially practicable level for practical pulp bleaching. Further, as shown in the graph plotted with white dots in Fig. 8, the quantity of Mn (III) generated by the FSM-MnP (as assayed at the absorbance at 270 nm) was stable throughout the bleaching, indicating that FSM-MnP had sufficient stability applicable to practical pulp bleaching process.

### (Example 9- Pulp bleaching with immobilized laccase)

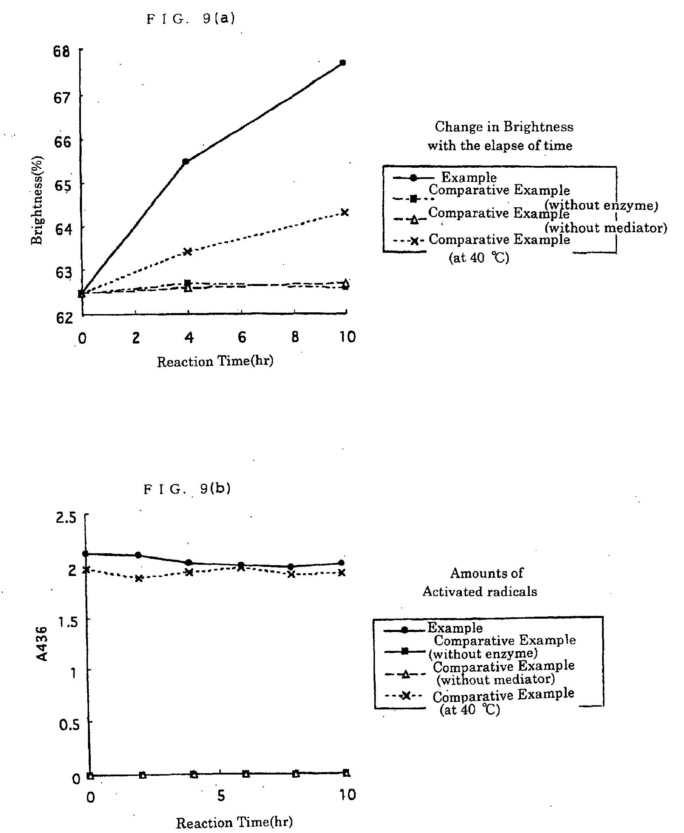

FSM with immobilized laccase of given activity units was packed into a column, which was kept warm at 40 °C, and then 50 mM acetate buffer (pH 4.5) containing 1 mM ABTS as the mediator was allowed to flow down continuously through the column. The reaction solution was continuously fed into a reaction tank where 1 % unbleached pulp kept warm at 70 °C was placed, for pulp bleaching. During this procedure, the concentration of active radicals derived from ABTS contained in the reaction solution was monitored, using the absorbance at 436 nm as the marker. The results are shown in Fig. 9(b), while Fig.9 (a) shows the assay results of the brightness of pulp drawn out of the reaction tank every given hours.

In Figs. 9 (a) and 9 (b), the mediator was activated by the enzyme to generate radicals, which reacted with pulp to increase the brightness of pulp in the present Example plotted with black round dots, while in a comparative example plotted with black square dots (example of FSM with no immobilized laccase as packed in column) or in a comparative example plotted with white triangle dots (example with no use of the mediator ABTS), activated radicals were never fed into the reaction tank, involving no increase of pulp brightness. In a comparative example (plots with "×") where the FSM-immobilized laccase column and the reaction tank were both kept warm at 40 °C, the bleaching rate was slower, compared with the Example.

Based on these results, it has been confirmed that the method of the invention is applicable to pulp bleaching using laccase and enabled efficient pulp bleaching in the same manner as in the use of MnP, by individually optimizing the reaction conditions for the enzyme reaction step and the pulp bleaching step.

### (Example 10- Pulp bleaching with immobilized lignin peroxidase)

FSM with immobilized lignin peroxidase was packed into a column, which was kept at 40 °C, and a succinate buffer (pH 4.5) containing 10 mM veratolyl alcohol and 0.4 mM hydrogen peroxide was allowed to flow down continuously through the column for 2-hour pulp bleaching by the same system as in Example 7. Consequently, the brightness in the Example was 63.3 %, while in the Comparative Example where FSM with no immobilized lignin peroxidase was packed in a column, the brightness was 61.9 %. It has already been confirmed that the difference between the Example and the Comparative Example is statistically significant by the significance test.

### [Examples in the Fifth Aspect of the Invention]

### (Example 11: Synthesis of mesoporous silica)

In a 100-ml beaker were individually placed 5.0 g (0.028 mol) of δ-Na₂Si₂O₅ (Kanemite) and 50 mL of ion exchange water, and the resulting mixture was agitated at about 25 °C for 3 hours, for cation exchange. After subsequent filtration of the aqueous solution, the precipitate δ-Na_{1.6}H_{0.4}Si₂O₅ was obtained. Into the precipitate was placed 50 mL of ion exchange water, for agitation to a homogenous dispersion, and the resulting dispersion was defined solution A. In a 100-ml Erlenmeyer flask were alternatively placed 3.0 g (0.0082 mol) of hexadecyltrimethylammonium bromide (HDTMA-Br) and 50 mL of ion exchange water, for agitation at 60 °C until the resulting mixture reached thorough transparency. Then, 5.0 g (0.025 mol) of triisopropylbenzene (TIPB) was added for vigorous agitation for 10 minutes, and the resulting mixture was defined solution B1. The solution A was transferred into a 250-ml three-neck flask. While vigorously agitating the solution A, the solution B was gradually added to the solution A, to raise the temperature to 80 °C. Then, the reaction continued at the constant temperature for 3 hours. While adjusting the pH of the reaction solution to 8.5 ± 0.1, using 2N hydrochloric acid, the reaction mixture was agitated for 3 hours, followed by immediate filtration and rinsing and filtration five times with ion exchange water of 200 mL. The filtered and separated product (white powder) was dried in air at 45 °C for 24 hours and subsequently sintered in an electric furnace at 550 °C for 6 hours, to obtain a mesoporous silica material of about 3.5 g, from which the template substance had been removed. The structure of the mesoporous silica material was confirmed by an X-ray diffraction apparatus (Rigaku RAD-B), and the diameter and surface area of the pores in the mesoporous silica material as well as the total pore volume therein were measured by a nitrogen gas adsorption apparatus (Autosorb mp-1). The details of the measurements are not described. The pore diameters were almost uniform, i.e. about 50 angstroms. The mesoporous silica material is designated as "FSM-50 carrier".

While the same solution A as described above was prepared, 4.0 g (0.01 mol) of dococyltrimethylammonium chloride (DTMA-C1) and 50 mL of ion exchange water were placed in a 100-ml Erlenmeyer flask for agitation at 60 °C, until the resulting mixture reached complete transparency. Then, 8.0 g (0.04 mol) of TIPB was added for vigorous agitation for 10 minutes, and the resulting solution was kept at 60 °C, which was defined solution B2. The solutions A and B2 were treated by the same procedures as for the solutions A and B1 in the Example 1-1, and the resulting product was dried in air and sintered in the same manner, to recover a mesoporous silica material of about 4.5 g. The structure confirmation of the mesoporous silica material and the measurements thereof were carried out as in Example 1-1. Although the details of the measurements are skipped, the pore diameters were almost uniform, i.e. about 70 angstroms. The mesoporous silica material is designated as "FSM-70 carrier".

By the same procedures as described above except that the amount of TIPB used during the preparation of the solution B was changed from 8.0 g (0.04 mol) to 16.0 g (0.08 mol), a mesoporous silica material of about 4.5 g was obtained. The structure confirmation of the mesoporous silica material and the measurements thereof were carried out as in Example 1-1. Although the details of the measurements are skipped, the pore diameters were almost uniform, i.e. about 90 angstroms. The mesoporous silica material is designated as "FSM-90 carrier".

### (Example 12: Preparation of FSM-MnP and the like)

Manganese peroxidase (MnP) obtained by culturing Phanerochaete crysosporium was adjusted to 0.2 mg/mL, using 50 mM sodium succinate buffer, pH 4.5. Then, about 70 mL each of the enzyme solution was added to 200 mg each of the "FSM-50 carrier", "FSM-70 carrier" and "FSM-90 carrier" powders. After mixing and kneading the resulting mixtures gradually at 4 °C for more than 16 hours, the mixtures were centrifuged and rinsed three times with deionized water, to obtain immobilized enzymes (individual MnP-conjugated FSM carriers). The three types of immobilized enzymes with different immobilization carriers (specifically, carriers with different pore diameters of the mesoporous silica material from each other) are collectively referred to as "FSM-MnP". When mentioning these enzymes separately, the FSM-MnP using the FSM-50 carrier is simply referred to as "FSM-50/MnP"; the FSM-MnP using the FSM-70 carrier is simply referred to as "FSM-70/MnP" and the FSM-MnP using the FSM-90 carrier is simply referred to as "FSM-90/MnP".

According to "Applied Biochemistry and Biotechnology, vol. 60, p. 1-17, 1996", alternatively, an immobilized enzyme was prepared by immobilizing the same manganese peroxidase on polymer beads as in the case of FSM-MnP, finally to the same enzyme quantity per weight as in the case above. The enzyme is referred to as "Emphaze" below, which is used as an immobilized enzyme for use in comparative examples. Using silica gel 5D (mean pore diameter of 190 angstroms) manufactured by Fuji Silysia, additionally, an immobilized enzyme was prepared by immobilizing the same manganese peroxidase as in the case of FSM-MnP, finally to the same enzyme quantity per weight as in the case above. The enzyme is referred to as "silica gel" below, which is used as an immobilized enzyme for use in comparative examples.

### (Example 13: FSM-MnP assessment)

### pH stability of FSM-MnP in the presence of oxidizing reagent

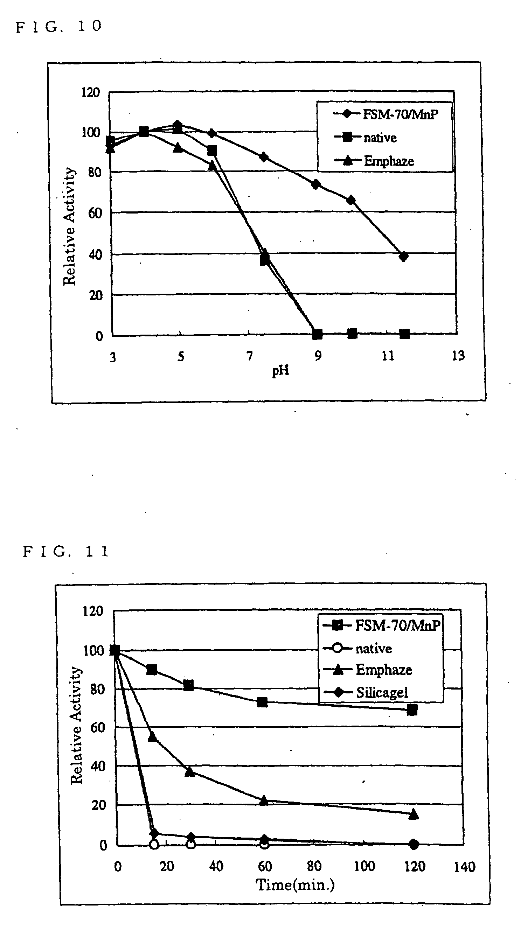

90 µL each of 50 mM buffers at various, different pHs was added to 5 mg of FSM-70/MnP, for treatment at 37 °C for one hour, and the solid therein was centrifuged, recovered and rinsed in 500 µL of ion exchange water. To the FSM-70/MnP was added 950 µL of 25 mM succinate buffer, pH 4.5, containing 0.5 mM MnSO₄ and 2 mM sodium oxalate as the substrates and 0.1 mM hydrogen peroxide, for reaction at 37 °C for 5 minutes. After subsequent 5-minute centrifugation, the absorbance of the supernatant was measured at 270 nm (trivalent manganese complex as the enzyme reaction product). Based on the measured value, the enzyme activity of FSM-70/MnP was assessed, which is plotted in Fig. 10 as relative numerical values taking the maximum activity value as 100 (expressed as "relative activity" in Fig. 10).

90 µL each of 50 mM buffers at various, different pHs was added to 4 units of non-immobilized manganese peroxidase, for treatment at 37 °C for one hour. Using 5 µl of the resulting mixture, the same reaction was performed to measure the absorbance in the same manner as described above, which is shown in Fig. 10. Further, 5 mg of the "Emphaze" was treated in the same manner as for FSM-70/MnP, for reaction. Then, the absorbance was measured in the same manner and is shown in Fig. 10. As shown in Fig. 10, almost no difference is observed between the non-immobilized manganese peroxidase expressed as "Native" in the figure and "Emphaze", but it is observed that the "FSM-70/MnP" exerts prominent pH stability in regions above pH 4, compared with both the enzymes.

### Stability of FSM-MnP over a period of time in the presence of oxidizing reagent

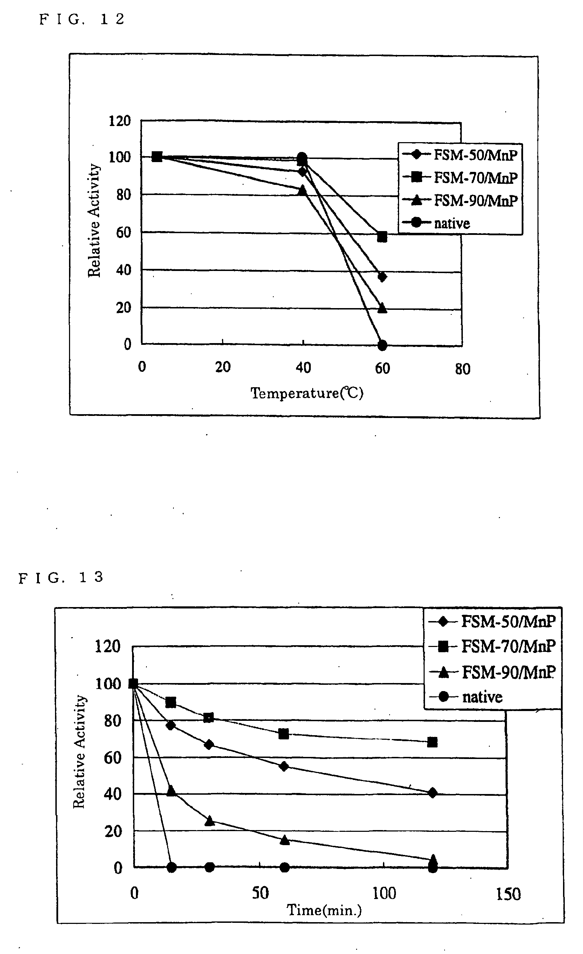

200 µL of 25 mM succinate buffer (pH 4.5) was added to 5 mg of FSM-70/MnP, for treatment at 60 °C for various periods of time, and then, followed by centrifugation of the solid therein, which was rinsed in 500 µL of deionized water. To the FSM-70/MnP was added 950 µL of 25 mM succinate buffer (pH 4.5) containing 0.5 mM MnSO₄ and 2 mM sodium oxalate as the substrates and 0.1 mM hydrogen peroxide, for reaction at 37 °C for 5 minutes. After subsequent 5-minute centrifugation, the absorbance of the supernatant was measured at 270 nm (trivalent manganese complex as the enzyme reaction product). Based on the measured value, the enzyme activity of FSM-70/MnP was assessed, which is plotted in Fig.11 as relative values (expressed as "relative activity" in Fig. 11) taking the maximum activity value as 100.

50 µL of 50 mM succinate buffer, pH 4.5 was added to 4 units of non-immobilized manganese peroxidase, for treatment at 60 °C for the various periods of time as mentioned above. Using 2.5 µl of the resulting mixture, the same reaction was performed to measure the absorbance in the same manner as described above, which is shown in Fig. 11. Further, 5 mg each of the "Emphaze" and the "silica gel" was treated in the same manner as in the case of the FSM-70/MnP, for reaction, and then, the absorbance was measured in the same manner and is shown in Fig. 11. As shown in Fig. 11, the stability of the non-immobilized manganese peroxidase expressed as "Native" in the figure became extremely low with the lapse of time, and the activities of the "Emphaze" and the "silica gel" were fairly rapidly reduced. In constrast, the "FSM-70/MnP" retained a relative activity of about 70 % even after 2 hours passed.

### Thermal stability of FSM-MnP in the presence of oxidizing reagent

200 µL of 25 mM succinate buffer (pH 4.5) was added to 5 mg each of FSM-50/MnP, FSM-70/MnP and FSM-90/MnP, for treatment at various temperatures for one hour, followed by centrifugation and further rinsing with 500 µL of deionized water. To these individual FSM-MnP was added 950 µL of 25 mM succinate buffer (pH 4.5) containing 0.5 mM MnSO₄ and 2 mM sodium oxalate as the substrates and 0.1 mM hydrogen peroxide, for reaction at 37 °C for 5 minutes. After subsequent 5-minute centrifugation, the absorbance of the supernatant was measured at 270 nm (trivalent manganese complex as the enzyme reaction product). Based on the measured value, the enzyme activities of the individual FSM-MnP were assessed, which is plotted in Fig.12 as relative values (expressed as "relative activity" in Fig. 12) taking the maximum activity value defined 100.

50 µL of 50 mM succinate buffer, pH 4.5 was added to 4 units of non-immobilized manganese peroxidase, for treatment at the same various temperatures as described above for one hour. Using 2.5 µL of the resulting mixture, the same reaction progressed to measure the absorbance in the same manner as described above, which is shown in Fig.12. As shown in Fig. 12, significant difference was observed in the relatively high temperature zone between the non-immobilized manganese peroxidase expressed as "Native" in the figure and the individual FSM-MnP. Among the individual FSM-MnP, the "FSM-70/MnP" with the pore size almost fitting to the enzyme diameter of manganese peroxidase was particularly excellent.

### Stability of FSM-MnP over a period of time in the presence of oxidizing reagent

200 µL of 200 mM succinate buffer (pH 4.5) was added to 5 mg each of FSM-50/MnP, FSM-70/MnP and FSM-90/MnP, for treatment at 60 °C for various periods of time, followed by centrifugation and rinsing with 500 µL of deionized water. To the individual FSM-MnP was added 950 µL of 25 mM succinate buffer (pH 4.5) containing 0.5 mM MnSO₄ and 2 mM sodium oxalate as the substrates and 0.1 mM hydrogen peroxide, for reaction at 37 °C for 5 minutes. After subsequent 5-minute centrifugation, the absorbance of the supernatant was measured at 270 nm (trivalent manganese complex as the enzyme reaction product). Based on the measured value, the enzyme activities of the individual FSM-MnP types were assessed, which is plotted in Fig.13 as relative values (expressed as "relative activity" in Fig. 13) taking the activity value as 100 at time 0.

50 µL of 50 mM succinate buffer, pH 4.5 was added to 4 units of non-immobilized manganese peroxidase, for treatment at 60 °C for the same various periods of time as described above. Using 2.5 µl of the resulting mixture, the same reaction was performed to measure the absorbance in the same manner as described above, which is shown in Fig. 13. As shown in Fig. 13, remarkable difference was observed in stability with the elapse of time between the non-immobilized manganese peroxidase expressed as "Native" in the figure and the individual FSM-MnP. Among the individual FSM-MnP, the "FSM-70/MnP" was particularly excellent.

### Stability of FSM-MnP against oxidizing reagent concentration

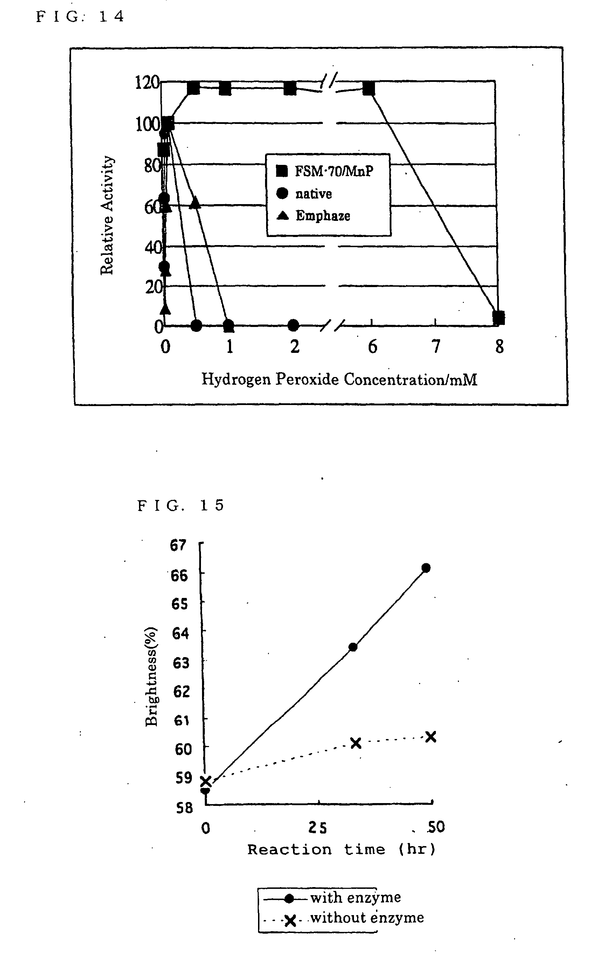

To 2 mg of FSM-70/MnP was added 950 µL of 25 mM succinate buffers (pH 4.5) containing a common combination of 0.5 mM MnSO₄ and 2 mM sodium oxalate as the substrates and various concentrations of hydrogen peroxide, for reaction at 37 °C for 5 minutes. After subsequent 5-minute centrifugation, the absorbance of the supernatant was measured at 270 nm (trivalent manganese complex as the enzyme reaction product). Based on the measured values, the enzyme activity of the FSM-70/MnP was assessed, which is plotted in Fig.14 as relative specific absorbance (expressed as "relative activity" in Fig. 14) to the absorbance at the hydrogen peroxide concentration of 0.

The buffer solutions containing the same various concentrations of hydrogen peroxide as described above were added to 3 units of non-immobilized manganese peroxidase, for the same reaction to measure the absorbance, which is shown in Fig. 14. Further, 2 mg of the "Emphaze" was treated in the same manner as in the case of the FSM-70/MnP, for reaction to measure the absorbance similarly, which is shown in Fig. 14. As shown in Fig. 14, the non-immobilized manganese peroxidase expressed as "Native" in the figure and the "Emphaze" were distinctly inactivated already at 0.5 mM hydrogen peroxide concentration, but the FSM-70/MnP retained effective activity even at the maximum 6 mM hydrogen peroxide concentration, compared with both the enzymes. Herein, no sufficient activity is shown in a low concentration range of hydrogen peroxide in the individual examples, which is due to the shortage of the absolute quantity of the oxidizing reagent but never indicates the reduction of the enzyme activity.

### (Example 14- Pulp bleaching)

FSM-70/MnP in slightly coarse granule was packed in a column, through which a buffer solution at 39 °C [50 mM malonate buffer (pH 4.5), 0.1 mM manganese sulfate, 0.05 % Tween 80, 0.1 mM hydrogen peroxide] was allowed to flow down at a flow rate of 200 mL/hr. for one hour, to continuously generate trivalent manganese. By continuously feeding the flow-through solution to a reaction tank placing therein 1 % unbleached pulp, pulp bleaching was effected. All these procedures were performed at 39 °C.

In a comparative example, the slightly coarse granule of the FSM-70 carrier with no immobilized enzyme was packed in a column, through which the same buffer solution was allowed to flow down, to perform the same pulp bleaching test using the flow-through solution.

These results are shown in graphs of Fig. 15, depicting the change of pulp brightness (%) over reaction time after the start of the bleaching test, where the pulp brightness was effectively improved in the present Example plotted with black dots, and there was a significant difference from the Comparative Example plotted with "×".

### [Examples in the Sixth to Eighth Aspects of the Invention]

### (Example 15- Pulp bleaching)

After Kraft cooking process, one kilogram of the resulting pulp was rinsed three times with 5 liters of distilled water to prepare unbleached pulp. Manganese peroxidase was purified from the liquid culture of P. chrysosporium BFC-1767 bacteria (ATCC 64314) by anion exchange chromatography.

Then, a test solution was prepared by respectively adding 50 mM acetylacetone, 50 mM malonic acid, 50 mM citric acid or 50 mM tartaric acid to 50 mM acetate buffer solution (pH 4.5) containing 0.1 mM manganese sulfate, 0.05 % Tween 80 and 0.5 M glucose.

After the unbleached pulp was added to 100 mL of the individual test solutions to a final 1 % pulp concentration, 100 units of manganese peroxidase and 5 units of glucose oxidase were added, for reaction under shaking at 37 °C and 50 rpm.

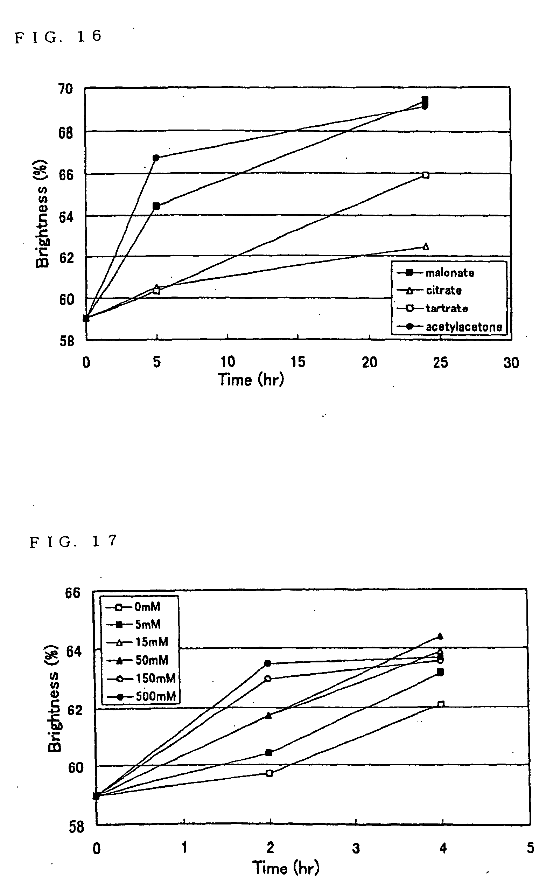

Using then the individual test solutions immediately after the start of the reaction, and 5 hours and 24 hours after the start of the reaction, pulp was filtered to prepare hand-made paper. The brightness of the resulting hand-made papers was measured according to JIS P-8123. The results are shown in Fig. 16. In the graphs of Fig. 16, the abscissa represents the time after the start of the reaction, while the ordinate represents the brightness (%).

As apparently shown in the results of Fig. 16, the pulp brightness can more rapidly be improved in the case using acetylacetone as a reaction promoter in the Example of the invention than in the case using malonic acid and other organic acids. The reason why the improvement of the pulp brightness from 5 hours to 24 hours after the start of the reaction has not any more increased may reside in the saturation of the effect on lignin decomposition (effect on the improvement of the brightness).

### (Example 16- Influence of acetylacetone concentration)

In relation to the test example added with 50 mM acetylacetone in Example 15, tests were conducted in the same manner, except that the quantity of acetylacetone added was changed to 0 mM, 5 mM, 15 mM, 150 mM and 500 mM. Herein, the brightness was measured immediately after the start of the reaction, and 2 hours and 4 hours after the start of the reaction. The results of those are shown in Fig. 17, including the example where 50 mM acetylacetone was added.

As apparently shown in the results of Fig. 17, the brightness of pulp is improved more rapidly as the concentration of acetylacetone added is higher. In the examples of a high concentration of acetylacetone added, herein, the pulp brightness was likely to be once improved rapidly but never be increased subsequently. The reason may reside in the saturation of the effect and in the enzyme inactivation with the high concentration of acetylacetone.

### (Example 17- Multi-step decomposition method)

The unbleached pulp prepared in Example 15 was suspended in aqueous 2.5 % sodium hydroxide solution to a final 1 % pulp concentration, for agitation under heating at 60 °C for 30 minutes (this alkali extraction process is referred to as E process). The unbleached pulp treated by the E process was subjected to the method for promoting decomposition in accordance with the invention, in the same manner as in Example 1 (this is referred to as M process).

Then, the E process and the M process were repeatedly carried out alternately to a total of 9 processes, and at the termination of the third, fifth, seventh and ninth processes of the E process, the resulting pulp was filtered to prepare hand-made paper and measured the brightness according to JIS P-8123.

In one comparative example, the same procedures were carried out except for no addition of acetylacetone in the M process, while in another comparative example, the same procedures were carried out except for no addition of both acetylacetone and manganese peroxidase in the M process. These comparative examples were also subjected to the test.

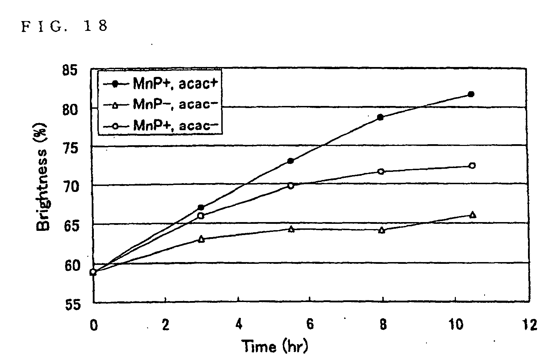

Those results are shown in Fig. 18. In Fig. 18, "MnP+, acac+ " represents the Example of the invention; "MnP+, acac-" represents the comparative example with no addition of both acetylacetone; and "MnP-, acac-" represents the comparative example with no addition of acetylacetone and manganese peroxidase. The abscissa in the figure does not show the order of the step, but shows the cumulative decomposition reaction period of time at each process. Further, each plot expresses the third, fifth, seventh and ninth processes sequentially from the left.

Fig. 18 indicates that the effect of improving pulp brightness is exerted more rapidly and larger in the Example than in the Comparative Examples and that in the Example, the saturation tendency of the effect never emerges until a high level of brightness is attained and a very high level of brightness exceeding 80 % is achieved finally.

### (Example 18- Enzyme immobilization)

Using a lamellar silicate Kanemite as a raw material and a template substance for pore formation, a mesoporous silica material with a uniform pore diameter of 50 angstroms (referred to as "FSM-70") was prepared by a given method.

Separately, manganese peroxidase prepared in Example 15 was adjusted to 5 mg/mL, using 10 mM sodium succinate acetate buffer solution, pH 4.5. Then, 5 mL of this manganese peroxidase solution was added to 200 mg of the FSM-70, for gradual mixing and kneading at 4 °C for 16 hours or more, to obtain manganese peroxidase immobilized on FSM-70 (referred to as "FSM-MnP").

### (Example 19- Separation between enzyme reaction and pulp bleaching reaction)

200 mL of 50 mM malonate buffer solution (pH 4.5) containing 10 mM manganese sulfate, 0.05 % Tween 80 and 0.1 mM hydrogen peroxide was passed at a flow rate of 8 mL/min. through a minicolumn packed with 3 mg of FSM-MnP, to recover the flow-through solution and prepare a solution of trivalent manganese ion.

To 50 mL of the solution was added pulp of a dry weight of 0.5 g in a comparative example, and pulp of a dry weight of 0.5 g and 0.5 M (final concentration) acetylacetone of the Example, for shaking at 70 °C for one hour. The elimination of the trivalent manganese ion was confirmed on the basis of the elimination of the absorbance at 270 nm, and thereafter, hand-made paper was prepared in the same manner as in Example 15 to measure the brightness.

Consequently, the brightness in the present Example was at 62.3 %, the improvement in brightness from unbleached pulp being 3.3 %. In the Comparative Example, the brightness was at 59.9 %, the improvement in brightness being 0.9 %. The aforementioned results indicate that according to the method for promoting the decomposition of a hydrophobic substrate in accordance with the invention, the oxidoreductase serves to provide trivalent manganese ion as an activated reaction mediator and the presence of the activated reaction mediator, reaction promoter and substrate is only required for the pulp bleaching reaction field.

Thus, the production of an activated reaction mediator with an oxidoreductase and the pulp bleaching reaction utilizing the activated reaction mediator are practiced on different fields, so that the method for promoting the decomposition of a hydrophobic substrate can thereby be progressed actively via the addition of a reaction promoter of a high concentration, while avoiding the enzyme inactivation with the reaction promoter.

### (Example 20- Paper quality test)

The pulp after the ninth process of "MnP+, acac+" in Example 17 and the pulp after the ninth process where the same procedures were carried out except for replacement of acetylacetone with malonic acid, were adjusted to a pulp concentration of about 0.3 %, using water, to prepare hand-made paper with the absolute dry weight of 60 g/m², using a square-shape sheet machine of one side of 250 mm in length, according to JIS P 8222. After the hand-made paper was pre-treated under conditions of 23 °C and 50 % r.h. according to JIS P 8111, the bursting strength was measured according to JIS P 8112.

Consequently, the specific bursting strength of the non-treated pulp was 6.8 kPa • m²/g, while that of the bleached pulp with addition of malonate was reduced to 6.5 kPa • m²/g. Alternatively, the specific bursting strength of the bleached pulp with addition of acetylacetone was never reduced from the value prior to bleaching, which remained 6.8 kPa • m²/g. The aforementioned results reveal that acetylacetone used as the reaction promoter decomposes only lignin as the hydrophobic substrate to promote bleaching, with no decomposition of the hydrophilic substrate cellulose, which works to avoid the deterioration of paper quality.

### [Examples in the Ninth to Eleventh Aspects of the Invention]

### (Example 21- Culture of SC21 strain and preparation of crude enzyme solution)

The Phanerochaete crysosporium SC21 strain (ATCC-64314) obtained from ATCC (American Type Culture Collection) was cultured, using the culture medium of T. K. Kirk et al. (Arch. Microbiol. 117, 277-285 (1978)) and M. H. Gold et al. (Arch. Biochem. Biophys. 234, 353-362 (1984)).

The spore was inoculated on a slant culture medium, for culture at 37 °C for about 2 weeks, to sufficiently form the spore. The formed spore was suspended in 5 mL of distilled water to prepare a spore suspension. One milliliter of the spore suspension was inoculated in 100 mL of a pre-culture medium, for stationary culture at 37 °C for 3 days.

The liquid pre-culture was homogenized for 30 seconds, to disperse the hyphae sufficiently, and then, 100 mL of the liquid pre-culture was inoculated in one liter of a liquid culture. After the liquid culture was subjected to rotary culture (50 mm, 150 rpm) at 37 °C for 3 days, veratolyl alcohol was added to the culture. Further, the inside of the flask was substituted with oxygen and pressurized (0.25 kgf/cm²), to continue the culture. Thereafter, the substitution with oxygen and pressurization in the same manner as described above, once a day, were continued until the termination of the culture.

When the color of the liquid culture turned reddish dark brown and the enzyme activity in the supernatant reached a sufficiently high level, the culture was terminated. Then, the bacterial cell was removed through a glass filter, to obtain a culture filtrate. To the resulting culture filtrate was added PEG4000 (5 %), and the resulting mixture was adjusted to pH 7.2 using sodium hydroxide, for gradual agitation in ice. The generated slime was removed through a glass filter, to obtain a crude enzyme solution.

### (Example 22- MnP purification)

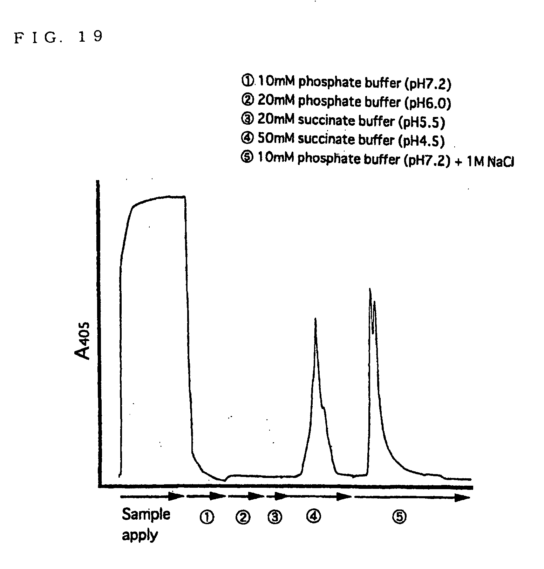

One liter of the crude enzyme solution obtained in Example 21 was adsorbed onto an anion exchange resin DEAE Sepharose Fast Flow column (manufactured by Pharmacia; 50 mm × 100 mm), which was then rinsed with a rinse buffer (10 mM sodium phosphate, pH 7.2). Subsequently, three types of buffers, namely elution buffer 1 (20 mM sodium phosphate, pH 6.0), elution buffer 2 (20 mM sodium succinate, pH 5.5) and elution buffer 3 (50 mM sodium succinate, pH 4.5) were sequentially passed through the column to elute MnP. MnP was detected by measuring the absorbance at 405 nm, which is unique to heme protein. The absorption peak at 405 nm satisfactorily coincides with the MnP activity and as shown in Fig. 19, MnP was eluted with the elution buffer 3. In Fig. 19, the rinse buffer, the elution buffer 1, the elution buffer 2 and the elution buffer 3 are expressed with 1, 2, 3 and 4, respectively. The eluted MnP active fraction was dialyzed against 10 mM sodium succinate buffer (pH 3.5) and then adsorbed onto a cation exchange column MonoS 5/5HR.

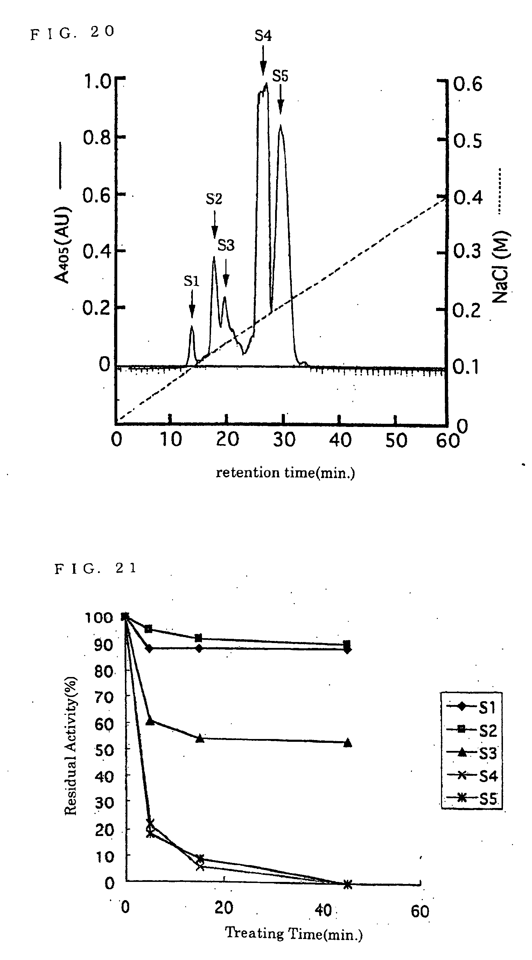

The adsorbed MnP was eluted on a 0 - 0.4 M linear concentration gradient of sodium chloride from the column, and as shown in Fig. 20, five peaks with absorption at 405 nm were separated. These peak fractions had MnP activity, indicating that five isozyme types with different properties exist. The isozymes contained in the active peaks are defined S1 S2, S3, S4 and S5 in the order of the short retention time.

### (Example 23- Analysis of molecular weight and isoelectric point of isozyme)

The molecular weight of MnP contained in each of the active fractions was analyzed by SDS-PAGE. A single band of a molecular weight of about 46,000 was observed in each of the active fractions, which indicates that the isozymes are isozymes having approximately equal molecular weights. Further, the isoelectric point of MnP contained in each of the active fractions was analyzed by isoelectric focusing using IEF PAGE mini (manufactured by TEFCO). It is revealed that the isoelectric points pIs of the five types of the isozymes are 4.3 for S1, 4.5 for S2, 4.7 for S3, 4.8 for S4 and 4.9 for S5 and that these isozymes are isozymes having different isoelectric points from each other.

### (Example 24- Enzyme stability)

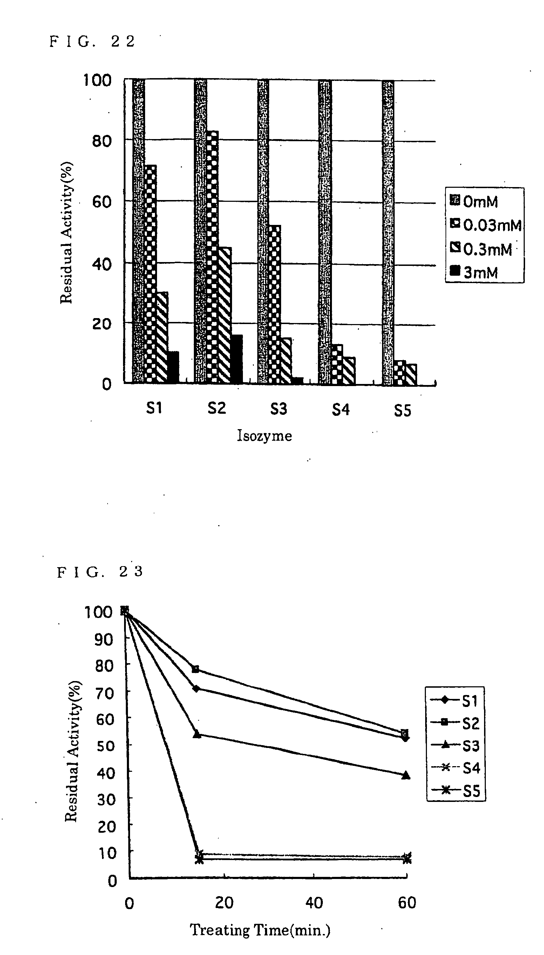

The isozymes were thermally treated in 50 mM succinate buffer (pH 4.5) at 50 °C for a given period of time, and the residual activities thereof were measured over a period of time to assess the thermal resistance thereof. The results are shown in Fig. 21. As apparently shown in Fig. 21, S4 and S5 had relatively poor thermal resistance, while S1 and S2 had very excellent thermal resistance. S3 had thermal resistance at an intermediate level among them.

Further, the isozymes were treated in the presence of hydrogen peroxide at concentrations of 0 mM, 0.03 mM, 0.3 mM and 3 mM at 37 °C for one hour, and the residual activities were subsequently measured. The results are shown in Fig. 22. As apparently shown in Fig. 22, S4 and S5 were almost inactivated in the presence of 0.03 mM hydrogen peroxide. In the presence of the same concentration of hydrogen peroxide, S1 and S2 were observed to have residual activities of 70 % or more, while S3 was observed to have a residual activity of 50 % or more. Additionally, these isozymes S1 to S3 were observed to have certain constant residual activities even in the presence of hydrogen peroxide at a concentration as high as 3 mM.

These results indicate that isozymes with identical functions sometimes have greatly different environmental resistance and that an enzyme or isozyme with great environmental resistance is of importance for industrial application.

### (Example 25- FSM-MnP preparation)

An active fraction containing each of the isozymes S1 to S5 was dialyzed sufficiently against 10 mM succinate buffer, and the resulting dialysate was added at a ratio of 50 U/mg to the FSM with a pore size of about 70 angstroms, followed by overnight agitation at 4 °C, to thereby immobilize MnP in the FSM to prepare FSM-MnP.

### (Example 26- Thermal resistance of FSM-MnP)

The FSM-MnP prepared by immobilizing each of the isozymes S1 to S5 in the manner described above was thermally treated in 50 mM succinate buffer (pH 4.5) at 60 °C for a given period of time, to measure the residual activity over a period of time and thereby assess the thermal resistance. The results are shown in Fig. 23. As apparently shown in the comparison between Fig. 23 and Fig. 21, the thermal resistance of the FSM-MnP reflects the thermal resistance of the immobilized isozyme.

### (Example 27- Pulp bleaching using FSM-MnP)

20 mg of the FSM-MnP prepared by immobilizing each of the isozymes S1 to S5 in the manner described above was packed in a column, through which a bleaching buffer [30 mM malonate buffer (pH 4.5), 10 mM manganese sulfate, 0.05 % Tween 80, 0.1 mM hydrogen peroxide] was allowed to continuously flow down to activate manganese. An enzyme reaction solution containing the activated manganese was fed into a reaction tank containing pulp at a concentration of 1 % for pulp bleaching.

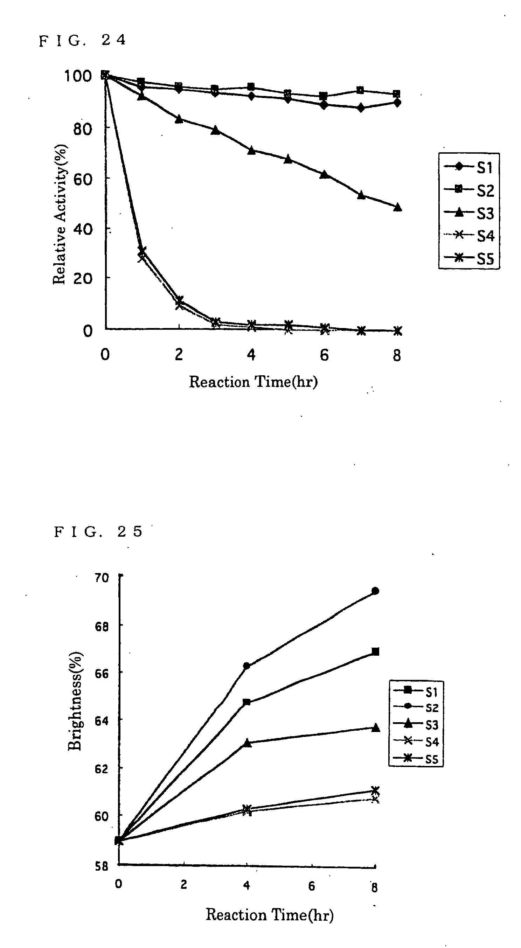

The change of the enzyme activity with the elapse of time was monitored by measuring the absorbance at 270 nm, and concurrently, a pulp sample drawn out of the reaction tank after a given time passed was used for preparing pulp paper, of which the brightness (%) was measured according to JIS P 8123, to assess the pulp bleaching activity of FSM-MnP. The results of the measurement of the change of the enzyme activity with the elapse of time are shown in Fig. 24, while Fig. 25 shows the results of the evaluation of the pulp bleaching activity.

As apparently shown in Fig. 24, the activity of the FSM-MnP with immobilized S1 or S2 at the termination of the 8-hour experiment was about 90 %, while the same activity of the FSM-MnP with immobilized S3 was about 50 %. However, the activity of the FSM-MnP with immobilized S4 or S5 was rapidly reduced after the start of the reaction and the residual activity after such one-hour reaction was about 30 % or less. After 2-hour reaction, the FSM-MnP was almost inactivated.

As shown in Fig. 25, the pulp brightness was increased with the reaction time in the case that the FSM-MnP with immobilized S1 or S2 was used. In the late reaction stage, the increment ratio was slowed down. After 8 hours passed, however, the brightness was increased by 8 points or 10 points, respectively. In the case that FSM-MnP with immobilized S3 was used, the increment of the brightness after 8 hours was 5 points. In the case that FSM-MnP with immobilized S4 or S5 was used, the increment of the brightness after 8 hours was at most about 2 points.

These results indicate that the use of MnP with higher stability is essential for practically realizing the pulp bleaching technique with MnP.

### Industrial Field of Utilization

As described above, the invention is effectively utilized for various enzyme reactions industrially useful in which mediators are involved, and for reactions using mediator activating means except for enzymes, particularly for lignin decomposition of pulp or decomposition of various persistent chemicals.

## Claims

1. A reaction method in which a mediator acting in an activated state thereof on a substrate to perform a predetermined reaction is charged as an active mediator in the activated state into a reaction system where the substrate exists.

2. A reaction method according to claim 1, which comprises prearranging an activation step of activating the mediator by an enzyme as mediator activating means and charging the active mediator activated by the activation step into the reaction system.

3. A reaction method according to claim 2, wherein the activation step is conducted while putting the enzyme and the mediator in contact to each other under mild conditions provided that the active mediator is a stable substance, and the reaction between the active mediator and the substrate is conducted under severe conditions to yield a high reaction efficiency.

4. A reaction method according to claim 1, wherein the mediator is charged into the reaction system as a highly active mediator in a highly activated state with more improved activity and/or stability than those of the active mediator in the activated state.

5. A reaction method according to claim 4, wherein the highly active mediator is in a more highly active and excited state than the active mediator is, or the highly active mediator is a high-order compound of a mediator at least including a complex.

6. A reaction method according to claim 1, 4 or 5, wherein the active mediator or the highly active mediator is prepared by enzymatic actions on the mediator, catalytic actions thereon, photoirradiation thereon, electromagnetic irradiation thereon, voltage loading thereon, or plasma preparation thereof.

7. A reaction method according to claim 2, 3 or 6, wherein the enzyme is an oxidoreductase and the substrate is lignin to be decomposed for pulp bleaching.

8. A reaction apparatus for practicing a reaction method according to claim 2 or 3, wherein the mediator activating means is in a form separable from the active mediator, and which further comprises an activation reaction field capable of performing the activation step, and a substrate reaction field being in communication through transfer means of the active mediator with the activation reaction field and capable of performing the reaction between the active mediator and the substrate.

9. An enzymatic method for decomposing an enzyme substrate with peroxidase as an oxidoreductase using an oxidizing reagent, wherein an immobilized enzyme prepared by immobilizing the oxidoreductase in a structure unit with structural stability and with a dimension approximately fitting to the size of the enzyme, is used to decompose the substrate in the presence of the oxidizing reagent within a concentration range allowable through the immobilization.

10. An enzymatic method for decomposing a substrate according to claim 9, wherein the substrate is a persistent chemical.

11. An enzymatic method for decomposing a substrate according to claim 10, wherein the persistent chemical is lignin.

12. An enzymatic method for decomposing a substrate according to claim 9, 10 or 11, wherein the structure unit is a pore in a mesoporous silica material.

13. A reaction promoter for use in a substrate decomposition reaction system with an oxidoreductase, wherein the reaction promoter has a diketone structure radicalizable on the basis of the action of the oxidoreductase and is water-soluble.

14. A reaction promoter according to claim 13, wherein the reaction promoter is acetylacetone.

15. A method for promoting the decomposition of a hydrophobic substrate, which comprises allowing an oxidoreductase, a reaction mediator, and a reaction promoter according to claim 13 or 14 to coexist in an aqueous solution where a hydrophilic substrate and a substrate being relatively hydrophobic thereto exist in mixture, and preferentially decomposing the hydrophobic substrate with the radicalized reaction promoter.

16. A method for promoting the decomposition of a hydrophobic substrate, which comprises activating a reaction mediator with an oxidoreductase, and adding the activated reaction mediator and a reaction promoter according to claim 13 or 14 to an aqueous solution where a hydrophilic substrate and a relatively hydrophobic substrate exist in mixture, to preferentially decompose the hydrophobic substrate with the radicalized reaction promoter.

17. A method for promoting the decomposition of a hydrophobic substrate according to claim 15 or 16, wherein the aqueous solution is pulp solution; the hydrophilic substrate is cellulose; and the relatively hydrophobic substrate is lignin.

18. A method for promoting the decomposition of a hydrophobic substrate according to claim 17, wherein the method for promoting the decomposition of a hydrophobic substrate is repeatedly carried out, in multiple steps, while intermediately interposing an alkali extraction treatment of pulp.

19. A method for promoting the decomposition of a hydrophobic substrate according to claim 17, 18 or 19, wherein the oxidoreductase is peroxidase selected from the group consisting of at least manganese peroxidase, horseradish peroxidase and lignin peroxidase, and the reaction mediator is manganese ion.

20. A method for decomposing a persistent chemical, which comprises allowing an oxidoreductase, a reaction mediator, and a reaction promoter according to claim 13 or 14 to coexist in an aqueous solution containing a persistent chemical, and decomposing the persistent chemical with the reaction promoter radicalized.

21. A method for decomposing a persistent chemical according to claim 20, wherein the persistent chemical is dioxin, polychlorobiphenyl (PCB) or an endocrine disruptor.

22. A manganese peroxidase enzyme solution exerting the following thermal resistance (1) and hydrogen peroxide resistance (2), as obtained as a liquid culture of an SC26 strain (deposited as ATCC-64314) as a mutant strain of Phanerochaete crysosporium generated via ultraviolet irradiation, or obtained from the liquid culture:
(1) the residual activity of the manganese peroxidase after thermal treatment at 50 °C for 45 minutes is 50 % or more;
(2) the residual activity of the manganese peroxidase in the presence of 0.3 mM hydrogen peroxide at 37 °C one hour later is 15 % or more.

23. A manganese peroxidase satisfying the following conditions (3) to (5):
(3) the manganese peroxidase is generated by an SC26 strain (deposited as ATCC-64314) as a mutant strain of Phanerochaete crysosporium generated via ultraviolet irradiation;
(4) the isoelectric point is within a range of 4.3 to 4.7;
(5) the residual activity of the manganese peroxidase after thermal treatment at 50 °C for 45 minutes is 50 % or more and the residual activity of the manganese peroxidase in the presence of 0.3 mM hydrogen peroxide at 37 °C one hour later is 15 % or more.

24. A manganese peroxidase according to claim 23, wherein the isoelectric point of the manganese peroxidase is 4.3; the residual activity of the manganese peroxidase after thermal treatment at 50 °C for 45 minutes is 90 % or more; and the residual activity of the manganese peroxidase in the presence of 0.3 mM hydrogen peroxide at 37 °C one hour later is 30 % or more.

25. A manganese peroxidase according to claim 23, wherein the isoelectric point of the manganese peroxidase is 4.5; the residual activity of the manganese peroxidase after thermal treatment at 50 °C for 45 minutes is 90 % or more; and the residual activity of the manganese peroxidase in the presence of 0.3 mM hydrogen peroxide at 37 °C one hour later is 45 % or more.

26. A manganese peroxidase according to claim 23, wherein the isoelectric point of the manganese peroxidase is 4.7; the residual activity of the manganese peroxidase after thermal treatment at 50 °C for 45 minutes is 50 % or more; and the residual activity of the manganese peroxidase in the presence of 0.3 mM hydrogen peroxide at 37 °C one hour later is 15 % or more.

## Amended claims

### Amended claims under Art. 19.1 PCT

**1.** A reaction method in which a mediator acting in an activated state thereof on a substrate to perform a predetermined reaction is charged as an active mediator in the activated state into a reaction system where the substrate exists.

**2.** A reaction method according to claim 1, which comprises prearranging an activation step of activating the mediator by an enzyme as mediator activating means and charging the active mediator activated by the activation step into the reaction system.

**3.** A reaction method according to claim 2, wherein the activation step is conducted while putting the enzyme and the mediator in contact to each other under mild conditions provided that the active mediator is a stable substance, and the reaction between the active mediator and the substrate is conducted under severe conditions to yield a high reaction efficiency.

**4.** A reaction method according to claim 1, wherein the mediator is charged into the reaction system as a highly active mediator in a highly activated state with more improved activity and/or stability than those of the active mediator in the activated state.

**5.** A reaction method according to claim 4, wherein the highly active mediator is in a more highly active and excited state than the active mediator is, or the highly active mediator is a high-order compound of a mediator at least including a complex.

**6.** A reaction method according to claim 1, 4 or 5, wherein the active mediator or the highly active mediator is prepared by enzymatic actions on the mediator, catalytic actions thereon, photoirradiation thereon, electromagnetic irradiation thereon, voltage loading thereon, or plasma preparation thereof.

**7.** A reaction method according to claim 2, 3 or 6, wherein the enzyme is an oxidoreductase and the substrate is lignin to be decomposed for pulp bleaching.

**8.** A reaction apparatus for practicing a reaction method according to claim 2 or 3, wherein the mediator activating means is in a form separable from the active mediator, and which further comprises an activation reaction field capable of performing the activation step, and a substrate reaction field being in communication through transfer means of the active mediator with the activation reaction field and capable of performing the reaction between the active mediator and the substrate.

**9.** (amended) A reaction method according to claim 2, 3 or 7, wherein the enzyme is an immobilized enzyme prepared by immobilizing peroxidase as an oxidoreductase in a structure unit with structural stability and with a dimension approximately fitting to the size of the enzyme.

**10.** (amended) A reaction method according to claim 9, wherein the structure unit is the pore in a mesoporous silica material.

**11.** (amended) A reaction method according to claim 1, 2, 3 or 7, wherein a reaction promoter having a diketone structure radicalizable on the basis of the action of the oxidoreductase and being water-soluble is present in a reaction system where the substrate exists.

**12.** (amended) A reaction method according to claim 11, wherein the reaction promoter is acetylacetone.

**13.** (amended) A reaction method according to claim 1, 2, 3 or 7, which comprises allowing an oxidoreductase, a reaction mediator, and a reaction promoter having a diketone structure radicalizable on the basis of the action of the oxidoreductase and being soluble in water to coexist in an aqueous solution where a hydrophilic substrate and a substrate hydrophobic relatively thereto exist in mixture, to preferentially decompose the hydrophobic substrate with the radicalized reaction promoter.

**14.** (amended) A reaction method according to claim 13, wherein the reaction promoter is acetylacetone.

**15.** (amended) A reaction method according to claim 1, 2, 3 or 7, which comprises activating a reaction mediator with an oxidoreductase, and adding the activated reaction mediator and a reaction promoter having a diketone structure radicalizable on the basis of the action of the oxidoreductase and being water-soluble to an aqueous solution where a hydrophobic substrate and a substrate hydrophobic relatively thereto exist in mixture, to preferentially decompose the hydrophobic substrate with the radicalized reaction promoter.

**16.** (amended) A reaction method according to claim 15, wherein the reaction promoter is acetylacetone.

**17.** (amended) A reaction method according to claim 13, 14, 15 or 16, wherein the aqueous solution is pulp solution; the hydrophilic substrate is cellulose; and the relatively hydrophobic substrate is lignin.

**18.** (amended) A reaction method according to claim 17, wherein the reaction method is carried out repeatedly, in multiple steps, while intermediately interposing an alkali extraction treatment of pulp.

**19.** (amended) A reaction method according to claim 15, 16, 17 or 18, wherein the oxidoreductase is peroxidase selected from the group consisting of at least manganese peroxidase, horseradish peroxidase and lignin peroxidase and the reaction mediator is manganese ion.

**20.** (amended) A reaction method according to claim 1, 2, 3 or 7, which comprises allowing an oxidoreductase, a reaction mediator, and a reaction promoter having a diketone structure radicalizable on the basis of the action of the oxidoreductase and being water-soluble to coexist in an aqueous solution containing a persistent chemical, to decompose the persistent chemical with the reaction promoter radicalized.

**21.** (amended) A reaction method according to claim 20, wherein the reaction promoter is acetylacetone.

**22.** (amended) A reaction method according to claim 20 or 21, wherein the persistent chemical is dioxin, polychlorobiphenyl (PCB) or an endocrine disruptor.

**23.** (amended) A reaction method according to claim 2, 3 or 7, wherein the enzyme is a manganese peroxidase enzyme solution exerting the following thermal resistance (1) and hydrogen peroxide resistance (2), as obtained as a liquid culture of an SC26 strain (deposited as ATCC-64314) as a mutant strain of Phanerochaete crysosporium, as generated via ultraviolet irradiation, or obtained from the liquid culture:
(1) the residual activity of the manganese peroxidase after thermal treatment at 50 °C for 45 minutes is 50 % or more;
(2) the residual activity of the manganese peroxidase in the presence of 0.3 mM hydrogen peroxide at 37 °C one hour later is 15 % or more.

**24.** (amended) A reaction method according to claim 2, 3 or 7, wherein the enzyme is a manganese peroxidase satisfying the following conditions (3) to (5):
(3) the manganese peroxidase generated by an SC26 strain (deposited as ATCC-64314) as a mutant strain of Phanerochaete crysosporium, as generated via ultraviolet irradiation;
(4) the isoelectric point within a range of 4.3 to 4.7;
(5) the residual activity of the manganese peroxidase after thermal treatment at 50 °C for 45 minutes is 50 % or more and the residual activity of the manganese peroxidase in the presence of 0.3 mM hydrogen peroxide at 37 °C one hour later is 15 % or more.

**25.** (amended) A reaction method according to claim 24, wherein the isoelectric point of the manganese peroxidase is 4.3; the residual activity of the manganese peroxidase after thermal treatment at 50 °C for 45 minutes is 90 % or more; and the residual activity of the manganese peroxidase in the presence of 0.3 mM hydrogen peroxide at 37 °C one hour later is 30 % or more.

**26.** (amended)A manganese peroxidase according to claim 24, wherein the isoelectric point of the manganese peroxidase is 4.5; the residual activity of the manganese peroxidase after thermal treatment at 50 °C for 45 minutes is 90 % or more; and the residual activity of the manganese peroxidase in the presence of 0.3 mM hydrogen peroxide at 37 °C one hour later is 45 % or more.

**27.** (added) A manganese peroxidase according to claim 24, wherein the isoelectric point of the manganese peroxidase is 4.7; the residual activity of the manganese peroxidase after thermal treatment at 50 °C for 45 minutes is 50 % or more; and the residual activity of the manganese peroxidase in the presence of 0.3 mM hydrogen peroxide at 37 °C one hour later is 15 % or more.

**28.** (added) An enzymatic method for decomposing an enzyme substrate with peroxidase as an oxidoreductase using an oxidizing reagent, wherein an immobilized enzyme prepared by immobilizing the oxidoreductase in a structure unit with structural stability and with a dimension approximately fitting to the size of the enzyme, is used to decompose the substrate in the presence of the oxidizing reagent within a concentration range allowable through the immobilization.

**29.** (added) An enzymatic method for decomposing a substrate according to claim 28, wherein the substrate is a persistent chemical.

**30.** (added) An enzymatic method for decomposing a substrate according to claim 29, wherein the persistent chemical is lignin.

**31.** (added) An enzymatic method for decomposing a substrate according to claim 28, 29 or 30, wherein the structure unit is the pore in a mesoporous silica material.

**32.** (added) A reaction promoter for use in a substrate decomposition reaction system of a substrate with an oxidoreductase, wherein the reaction promoter has a diketone structure radicalizable on the basis of the action of the oxidoreductase and is water-soluble.

**33.** (added) A reaction promoter according to claim 32, wherein the reaction promoter is acetylacetone.

**34.** (added) A method for promoting the decomposition of a hydrophobic substrate, which comprises allowing an oxidoreductase, a reaction mediator, and a reaction promoter according to claim 32 or 33 to coexist in an aqueous solution where a hydrophilic substrate and a substrate hydrophobic relatively thereto exist in mixture, to preferentially decompose the hydrophobic substrate with the radicalized reaction promoter.

**35.** (added) A method for promoting the decomposition of a hydrophobic substrate, which comprises activating a reaction mediator with an oxidoreductase, and adding the activated reaction mediator and a reaction promoter according to claim 32 or 33 to an aqueous solution where a hydrophilic substrate and a substrate hydrophobic relatively thereto exist in mixture, to preferentially decompose the hydrophobic substrate with the radicalized reaction promoter.

**36.** (added) A method for promoting the decomposition of a hydrophobic substrate according to claim 34 or 35, wherein the aqueous solution is pulp solution; the hydrophilic substrate is cellulose; and the relatively hydrophobic substrate is lignin.

**37.** (added) A method for promoting the decomposition of a hydrophobic substrate according to claim 36, wherein the method for promoting the decomposition of a hydrophobic substrate is repeatedly carried out, in multiple steps, while intermediately interposing an alkali extraction treatment of pulp.

**38.** (added) A method for promoting the decomposition of a hydrophobic substrate according to claim 35, 36 or 37, wherein the oxidoreductase is peroxidase selected from the group consisting of at least manganese peroxidase, horseradish peroxidase and lignin peroxidase and the reaction mediator is manganese ion.

**39.** (added) A method for decomposing a persistent chemical, which comprises allowing an oxidoreductase, a reaction mediator and a reaction promoter according to claim 32 or 33 to coexist in an aqueous solution containing a persistent chemical, to decompose the persistent chemical with the reaction promoter radicalized.

**40.** (added) A method for decomposing a persistent chemical according to claim 39, wherein the persistent chemical is dioxin, polychlorobiphenyl (PCB) or an endocrine disruptor.

**41.** (added) A manganese peroxidase enzyme solution exerting the following thermal resistance (1) and hydrogen peroxide resistance (2), as obtained as a liquid culture of an SC26 strain (deposited as ATCC-64314) as a mutant strain of Phanerochaete crysosporium generated via ultraviolet irradiation, or obtained from the liquid culture:
(1) the residual activity of the manganese peroxidase after thermal treatment at 50 °C for 45 minutes is 50 % or more;and
(2) the residual activity of the manganese peroxidase in the presence of 0.3 mM hydrogen peroxide at 37 °C one hour later is 15 % or more.

**42.** (added) A manganese peroxidase satisfying the following conditions (3) to (5):
(3) the manganese peroxidase is generated by an SC26 strain (deposited as ATCC-64314) as a mutant strain of Phanerochaete crysosporium generated via ultraviolet irradiation;
(4) the isoelectric point is within a range of 4.3 to 4.7;and
(5) the residual activity of the manganese peroxidase after thermal treatment at 50 °C for 45 minutes is 50 % or more and the residual activity of the manganese peroxidase in the presence of 0.3 mM hydrogen peroxide at 37 °C one hour later is 15 % or more.

**43.** (added) A manganese peroxidase according to claim 42, wherein the isoelectric point of the manganese peroxidase is 4.3; the residual activity of the manganese peroxidase after thermal treatment at 50 °C for 45 minutes is 90 % or more; and the residual activity of the manganese peroxidase in the presence of 0.3 mM hydrogen peroxide at 37 °C one hour later is 30 % or more.

**44.** (added) A manganese peroxidase according to claim 42, wherein the isoelectric point of the manganese peroxidase is 4.5; the residual activity of the manganese peroxidase after thermal treatment at 50 °C for 45 minutes is 90 % or more; and the residual activity of the manganese peroxidase in the presence of 0.3 mM hydrogen peroxide at 37 °C one hour later is 45 % or more.

**45.** (added) A manganese peroxidase according to claim 42, wherein the isoelectric point of the manganese peroxidase is 4.7; the residual activity of the manganese peroxidase after thermal treatment at 50 °C for 45 minutes is 50 % or more; and the residual activity of the manganese peroxidase in the presence of 0.3 mM hydrogen peroxide at 37 °C one hour later is 15 % or more.

Statement under Art. 19.1 PCT
Claims 9 to 27 have been added as subclaims of the invention relating to thereaction method. Claims 9 and 10 include the limitation relating to the use of the immobilized oxidoreductase; claims 11 and 12 include the limitation relating to the use of the reaction promoter; claims 13 to 19 include the limitation relating to the reaction system where a hydrophilic substrate and a hydrophobic substrate coexist in mixture; claims 20 to 22 include the limitation such that the substrate is a persistent chemical; and claims 23 to 27 include the limitation of the enzyme type and activity.

Claims 28 to 45 are substantially the same as original claims 9 to 26, except that the claim numbers have been changed because new claims 9 to 27 have been added.
